# FASCICULE DE BREVET EUROPEEN

(11) **EP 4 068 402 B1**
(45) Date de publication et mention de la délivrance du brevet: **22.03.2023**
(21) Numéro de dépôt: 21315055.0
(22) Date de dépôt: 29.03.2021
(51) Int. Cl.: H10N 30/30

(54) **MODULE À EXTRACTION OPTIMISÉE DE L'ÉNERGIE ISSUE D'UN TRANSDUCTEUR PIÉZOÉLECTRIQUE, NOTAMMENT POUR RECHARGER LA BATTERIE D'UN DISPOSITIF MÉDICAL IMPLANTABLE TEL QU'UNE CAPSULE CARDIAQUE AUTONOME LEADLESS**
MODUL MIT OPTIMIERTER ENERGIEENTNAHME AUS EINEM PIEZOELEKTRISCHEN WANDLER, INSBESONDERE ZUM AUFLADEN DER BATTERIE EINES IMPLANTIERBAREN MEDIZINISCHEN GERÄTS WIE EINER LEITUNGSLOSEN AUTONOMEN HERZKAPSEL
MODULE WITH OPTIMISED EXTRACTION OF THE ENERGY FROM A PIEZOELECTRIC TRANSDUCER, IN PARTICULAR FOR RECHARGING THE BATTERY OF AN IMPLANTABLE MEDICAL DEVICE SUCH AS A LEADLESS AUTONOMOUS CARDIAC CAPSULE

(43) Date de publication de la demande: 05.10.2022
(73) Titulaire: Cairdac, 92160 Antony (FR)
(72) Inventeur: Makdissi, Alaa, 75011 Paris (FR); Nguyen-Dinh, An, 37520 La Riche (FR)
(74) Mandataire: Dupuis-Latour, Dominique

(56) Documents cités:
- US-A1- 2014 056 043
- FANG SHENGHENG ET AL: "An Efficient Piezoelectric Energy Harvesting Circuit With Series-SSHI Rectifier and FNOV-MPPT Control Technique", IEEE TRANSACTIONS ON INDUSTRIAL ELECTRONICS, IEEE SERVICE CENTER, PISCATAWAY, NJ, USA, vol. 68, no. 8, 10 juillet 2020 (2020-07-10), pages 7146-7155, XP011852436, ISSN: 0278-0046, DOI: 10.1109/TIE.2020.3007054 [extrait le 2021-04-30]
- LI SHUO ET AL: "A Piezoelectric Energy-Harvesting System With Parallel-SSHI Rectifier and Integrated Maximum-Power-Point Tracking", IEEE SOLID-STATE CIRCUITS LETTERS, IEEE, vol. 2, no. 12, 1 décembre 2019 (2019-12-01), pages 301-304, XP011762062, DOI: 10.1109/LSSC.2019.2951394 [extrait le 2019-12-20]

## Description

### Domaine de l'invention

L'invention concerne les récupérateurs d'énergie, également dénommés "harvesters" ou "scavengers", qui recueillent l'énergie mécanique résultant de mouvements divers qu'ils subissent et convertissent cette énergie mécanique en énergie électrique.

Elle concerne plus spécifiquement les récupérateurs de type dit "PEH" (*Piezoelectric Energy Harvester*), qui utilisent comme transducteur mécano-électrique une lame piézoélectrique oscillante couplée à une masse mobile inertielle.

L'invention sera décrite plus particulièrement dans une application de ces récupérateurs d'énergie à des dispositifs médicaux autonomes, notamment les dispositifs de type capsule implantable autonome, en particulier ceux de ces dispositifs qui sont destinés à être implantés dans une cavité cardiaque.

Cette application, si elle est particulièrement avantageuse, ne doit toutefois pas être considérée comme limitative de l'invention, dont les enseignements peuvent être appliqués à de nombreux autres types de dispositifs autonomes incorporant un récupérateur d'énergie de type PEH, que ces dispositifs soient implantables ou non, qu'il s'agisse de dispositifs médicaux ou non.

### Description de la technique antérieure

Dans le domaine des implants médicaux, les récents progrès en matière de miniaturisation de dispositifs actifs et les progrès des sciences du vivant permettent dorénavant le développement d'une grande variété de systèmes miniaturisés implantables totalement autonomes, à des fins de surveillance, de diagnostic ou de traitement. Ces dispositifs mettent en oeuvre des procédures d'implantation moins invasives, plus de confort, des performances accrues et souvent ouvrent l'accès à des nouveaux types de diagnostics et traitements.

Lorsqu'elle est appliquée au domaine des implants médicaux, l'invention concerne plus précisément ceux de ces implants qui incorporent un système d'autoalimentation comprenant un récupérateur d'énergie mécanique associé à un organe de stockage d'énergie intégré tel qu'une batterie rechargeable ou une capacité à hautes performances.

L'un des aspects critiques de ces dispositifs miniaturisés est celui de l'autonomie électrique. La durée de vie d'un tel implant étant d'environ 8 à 10 ans, compte tenu des très faibles dimensions il n'est pas possible d'utiliser une batterie conventionnelle, même à forte densité.

Le récupérateur PEH pallie cet inconvénient en recueillant l'énergie mécanique résultant des mouvements divers subis par le corps du dispositif implanté. Ces mouvements peuvent avoir pour origine un certain nombre de phénomènes se produisant par exemple au rythme des battements cardiaques tels que les secousses périodiques de la paroi sur laquelle est ancré l'implant, les vibrations des tissus cardiaques liées entre autres aux fermetures et ouvertures des valves cardiaques, ou encore les variations de débit du flux sanguin dans le milieu environnant, qui sollicitent l'implant et le font osciller au rythme des variations de débit. L'énergie mécanique recueillie par le PEH est convertie en énergie électrique (tension ou courant) au moyen d'un transducteur mécano-électrique approprié, pour assurer la recharge de l'organe de stockage d'énergie et l'alimentation des divers circuits et capteurs du dispositif. Ce système d'alimentation permet au dispositif de fonctionner en parfaite autonomie électrique pendant toute sa durée de vie.

Cette technique de récupération d'énergie est particulièrement bien adaptée à l'alimentation des capsules autonomes implantées dépourvues de toute liaison physique à un dispositif distant. Ces capsules sont dénommées pour cette raison "capsules leadless", pour les distinguer des électrodes ou capteurs disposés à l'extrémité distale d'une sonde (*lead*) parcourue sur toute sa longueur par un ou plusieurs conducteurs reliés à un générateur connecté à l'extrémité opposée, proximale.

L'invention n'est toutefois pas limitée à un type particulier de capsule, ni même d'implant leadless, et elle est applicable indifféremment à de nombreux autres types de dispositifs médicaux implantables, quelle que soit leur destination fonctionnelle, cardiaque ou autre, médicale ou non.

Dans le cas d'une application cardiaque, la capsule leadless surveille en continu le rythme du patient et délivre si nécessaire au coeur des impulsions électriques de stimulation, de resynchronisation et/ou de défibrillation en cas de troubles du rythme détecté par la capsule. La capsule comprend divers circuits électroniques, capteurs, etc. ainsi que des moyens émetteurs/récepteurs de communication sans fil pour l'échange de données à distance, le tout étant intégré dans un corps de très petites dimensions qui puisse être implanté dans des sites difficilement accessibles ou laissant peu d'espace, tels que l'apex du ventricule, la paroi interne de l'oreillette, etc.

Le document US2014056043, la publication par Shengheng Fang intitulée "An Efficient Piezoelectric Energy Harvesting Circuit With Series-SSHI Rectifier and FNOV-MPPT Control Technique" publiée dans le journal IEEE Transactions on industrial electronics le 10.07.2020 et la publication par Shuo Li intitulée "A Piezoelectric Energy-Harvesting System With Parallel-SSHI Rectifier and Integrated Maximum-Power-Point Tracking" publiée dans le journal IEEE Solid-State Circuits Letters le 01.12.2019 divulguent respectivement un système de récupération d'énergie comprenant un convertisseur régulateur et un contrôleur de MPPT.

Le WO 2019/001829 A1 (Cairdac) décrit un exemple d'une telle capsule leadless intracardiaque.

L'invention concerne plus précisément les capsules ou dispositifs implantables analogues dont le récupérateur d'énergie est du type PEH, c'est-à-dire utilisant un transducteur piézoélectrique ou "PZT" (*PieZoelectric Transducer*) et un ensemble pendulaire inertiel. L'ensemble pendulaire inertiel comprend une masse mobile logée dans le corps de la capsule, dite "masse sismique" ou "masse inertielle", qui est entrainée au gré des mouvements de la capsule soumise en permanence aux diverses sollicitations externes décrites plus haut, appliquées à la fréquence de récurrence des cycles cardiaques, de l'ordre de 60 à 120 bpm (battements par minute) soit 1 à 2 Hz. Après chacune de ces sollicitations, la masse inertielle, qui est couplée à un élément élastiquement déformable, oscille à une fréquence propre d'oscillation libre, typiquement de l'ordre de 20 Hz. L'énergie mécanique de l'oscillation est convertie en énergie électrique par le PZT sollicité de façon cyclique et alternative en flexion de manière à engendrer au sein du matériau qui le constitue des charges électriques, qui sont récupérées en surface du composant pour être utilisées par le système d'autoalimentation de la capsule leadless. Le PZT peut notamment se présenter sous la forme d'une lame encastrée à l'une de ses extrémités et couplée à la masse inertielle à son autre extrémité, qui est libre.

Le signal électrique en sortie du PZT est délivré à un circuit de gestion de l'alimentation de la capsule, qui redresse et régule le signal électrique pour délivrer en sortie une tension ou un courant continus stabilisés permettant d'assurer l'alimentation des divers circuits électroniques et capteurs de la capsule, ainsi que la recharge de l'organe de stockage d'énergie. Un tel récupérateur d'énergie de type PEH est notamment décrit dans le US 3,456,134 A (Ko) et dans le WO 2019/001829 A1 précité.

Le signal électrique produit par la lame piézoélectrique ne peut toutefois pas être directement utilisé pour alimenter les divers circuits électroniques et capteurs de la capsule et recharger l'organe de stockage d'énergie (batterie rechargeable ou autre moyen de stockage tel qu'un condensateur à hautes performances, ci-après désignés par le terme générique de "batterie").

Pour cela, le signal électrique alternatif oscillant amorti recueilli aux bornes de la lame piézoélectrique est délivré en entrée à un circuit de gestion de l'alimentation de la capsule, habituellement désigné "PMU" (*Power Management Unit*). Ce circuit redresse et régule le signal électrique alternatif oscillant et délivre en sortie une tension ou un courant stabilisés permettant d'assurer à la fois la recharge de la batterie et l'alimentation des circuits applicatifs de l'implant.

L'utilisation d'un PZT comme source d'énergie rend cette gestion de l'alimentation particulièrement complexe.

Une première difficulté réside dans le fait que le signal électrique alternatif oscillant amorti délivré par le PZT équivaut, du point de vue électrique, à un générateur fortement non linéaire, avec une énergie instantanée délivrée variable et inconnue, car dépendant fortement de l'amplitude des déformations de la lame, elles-mêmes très dépendantes des sollicitations externes qui entraînent l'ensemble pendulaire en vibration.

Une deuxième difficulté tient au fait que, également du point de vue électrique, le PZT peut être assimilé à une source de courant alternatif d'impédance interne élevée, essentiellement capacitive. De ce fait, outre l'énergie extraite du PZT pour délivrer une alimentation, la tension produite par les oscillations de la lame charge la capacité interne du PZT, et il est nécessaire de décharger de la façon la plus appropriée les charges accumulées dans cette capacité interne afin de minimiser les pertes par autodécharge au sein du PZT.

Ces contraintes, qui sont spécifiques à l'emploi d'un PZT comme source primaire d'énergie, ont conduit pour maximiser la puissance extraite par le PEH à proposer de nombreuses configurations de circuits dédiés, plus complexes qu'un simple redresseur double-alternance FBR (*Full-Bridge Rectifier*). Ainsi, le circuit redresseur recevant la tension variable générée par le PZT, également dénommé "circuit d'interface" du PEH, peut être un circuit de type :
SSHI (*Synchronized Switch Harvesting on Inductor*),
SSHI série S-SSHI (*Serial SSHI*) ou parallèle P-SSHI (*Parallel SSHI*), à inductance ou à condensateurs, ou
SECE (*Synchronous Electric-Charge Extraction*) à inductance ou à condensateurs, ou encore
SSPB (*Single-Supply Pre-Biasing*) à inductance.

Ces circuits d'interface sont efficaces mais, du fait de leur complexité, entraînent une consommation propre non négligeable, qui vient donc au détriment de l'autonomie de l'implant. De plus, la taille d'une inductance ayant un facteur de qualité élevé, ou d'un condensateur de capacité suffisante, n'est pas compatible avec l'extrême miniaturisation requise par un dispositif implantable tel qu'une capsule leadless.

Il a par ailleurs été proposé une configuration de PEH dans lequel le circuit d'interface du PEH (redresseur FBR ou circuit plus complexe tel que SSHI ou autre) charge un condensateur intermédiaire de lissage monté en entrée d'un convertisseur continu/continu survolteur/dévolteur (*buck-boost*) délivrant en sortie une tension stabilisée permettant d'assurer la recharge d'une batterie et l'alimentation des circuits électriques applicatifs en aval de la batterie.

Un tel circuit d'interface à condensateur intermédiaire de lissage est par exemple décrit par G. K. Ottman et al., Adaptive Piezoelectric Energy Harvesting Circuit for Wireless Remote Power Supply, IEEE Transactions on Power Electronics, 17(5):669-676, Sept. 2002, ainsi que dans les US 8 026 650 B2 et US 2005/285728 A1.

Dans cet agencement connu, la tension du condensateur de lissage intermédiaire est surveillée en permanence, et il est déchargé de manière contrôlée afin de maximiser la puissance transférée en aval aux circuits applicatifs. La puissance transférée est toutefois fortement dépendante des performances et des pertes internes du convertisseur *buck-boost,* de sorte que l'énergie transférée depuis le condensateur intermédiaire varie en permanence en fonction du point de fonctionnement instantané du convertisseur, qui devra être adapté en permanence par une boucle de régulation tenant compte de ces variations.

Indépendamment de ces difficultés particulières de mise en oeuvre, les circuits PEH de ce type déjà proposés sont en général basés sur l'hypothèse que la lame vibre sous l'effet d'une source de vibrations qui est un signal harmonique, ou proche, de la fréquence de résonance du PZT, et/ou qu'elle vibre sous l'effet d'une impulsion qui induit une vibration harmonique propre du PZT.

Cette hypothèse correspond à une source de vibrations constituée de sollicitations de relativement faible amplitude mais très fréquentes. Mais dans une application à la stimulation cardiaque, cette hypothèse n'est pas vérifiée. En effet, les sollicitations mécaniques qui font vibrer le PZT ont pour origine des phénomènes se produisant typiquement au rythme des battements cardiaques (secousses de la paroi sur laquelle est ancré l'implant, variations de débit du flux sanguin qui font osciller l'implant, etc.). Ces sollicitations sont assimilables à des vibrations impulsionnelles de très forte amplitude, à une fréquence (de l'ordre de 1 à 2 Hz) qui est très inférieure à la fréquence propre d'un PZT (typiquement de l'ordre de 15 à 40 Hz).

Ces amplitudes élevées vont induire aux bornes du PZT une tension élevée, qui peut couramment atteindre plusieurs dizaines de volts, très supérieure à la tension de recharge de la microbatterie de l'implant, de l'ordre de quelques volts. Ces fortes amplitudes peuvent même s'accroître considérablement lors des périodes de stress ou d'effort du patient, avec des variations de tension instantanées très importantes aux bornes du PZT, pouvant typiquement atteindre ± 60 V en circuit ouvert. On constate alors que les circuits d'interface complexes décrits dans la littérature et présentés comme particulièrement bien adaptés aux récupérateurs de type PEH (circuits de type SSHI, SECE, SSPB, etc.), même combinés à un étage survolteur/dévolteur (*buck-boost*), ont dans ces circonstances une efficacité médiocre, avec dans les cas extrêmes un rendement de conversion qui n'est guère plus élevé que celui d'un simple redresseur FBR à pont de diodes.

Or le rendement d'extraction de puissance est un aspect particulièrement critique dans le cas d'un implant autonome : si par exemple les circuits applicatifs de l'implant requièrent une puissance de 5 µW et que le PEH ne délivre que 4,8 µW (c'est-à-dire seulement 4 % au-dessous de la puissance nominale requise), l'implant sera défaillant à court terme. De fait, toute augmentation du rendement du PEH, même minime, peut être essentielle pour garantir que l'implant fonctionnera correctement sur toute sa durée de vie et en toutes circonstances.

De plus, outre les aspects électriques et de rendement d'extraction que l'on vient d'exposer, la mise en oeuvre des circuits PEH connus est souvent incompatible avec les impératifs technologiques propres aux implants cardiaques.

Par exemple, une interface de type S-SSHI à inductance génère des courants élevés (de l'ordre de 160 mA pour une inductance de 1 mH ou de 320 mA pour 220 µH), qui sont incompatibles avec les microbatteries utilisées par les implants de type capsule leadless : ces microbatteries, qui ont une capacité de l'ordre de 1 mAh ou 2 mAh seulement, doivent être en principe rechargées à un courant de 1 mA ou 2 mA, en tout état de cause inférieur à 5 mA. Or, si l'on prévoit un condensateur tampon afin absorber les pics de courant et réduire le courant de charge à une valeur compatible avec la faible capacité de la batterie, l'encombrement supplémentaire important de ce condensateur ira à l'encontre des impératifs de miniaturisation extrême des implants cardiaques - le circuit de recharge devenant alors à lui seul plus volumineux que la batterie.

Le besoin existe donc de disposer, au sein notamment d'un implant cardiaque, d'un circuit convertisseur statique permettant de (i) maximiser la puissance extraite d'un PZT soumis à des sollicitations de forte amplitude et faible fréquence de type battements cardiaques pour (ii) délivrer une tension continue et stabilisée à une batterie et aux circuits électriques divers de l'implant, en minimisant les pertes de conversion, et (iii) en respectant les contraintes de miniaturisation extrême propres aux implants médicaux, tout particulièrement les capsules leadless.

### RÉSUMÉ DE L'INVENTION

Le but de l'invention est de proposer une configuration de PEH qui pallie les multiples inconvénients et contraintes exposés ci-dessus.

À cet effet, l'invention propose un module de récupération d'énergie, comprenant de manière en elle-même connue un ensemble pendulaire soumis à des sollicitations externes appliquées au module, l'ensemble pendulaire comprenant une lame élastiquement déformable en flexion suivant au moins un degré de liberté, avec une extrémité encastrée et une extrémité opposée libre couplée à une masse inertielle. La lame est une lame piézoélectrique formant un transducteur mécanoélectrique apte à convertir une énergie mécanique produite par des oscillations de l'ensemble pendulaire en un signal électrique alternatif oscillant recueilli par des électrodes de la lame. Un circuit de gestion d'alimentation redresse et régule le signal recueilli par les électrodes, pour délivrer en sortie une tension ou un courant continus d'alimentation stabilisés.

Ce circuit de gestion d'alimentation comprend : un circuit d'interfaçage avec la lame piézoélectrique, recevant en entrée le signal électrique alternatif oscillant délivré par la lame piézoélectrique et délivrant en sortie un signal redressé comprenant une série de pulsations à une fréquence multiple de la fréquence d'oscillation de l'ensemble pendulaire ; en sortie du circuit d'interfaçage, un condensateur tampon chargé par les pulsations successives délivrées par le circuit d'interfaçage ; un convertisseur régulateur apte à convertir un courant de décharge du condensateur tampon en ladite tension ou courant continus d'alimentation stabilisés ; et un circuit de pilotage du convertisseur régulateur, comprenant un étage d'asservissement de type *Maximum Power-Point Tracking*, MPPT, en fonction d'une estimée d'une puissance extraite de la lame piézoélectrique.

De façon caractéristique de l'invention, l'étage d'asservissement MPPT est commandé par une valeur courante d'un rapport cyclique, direct ou inverse, des pulsations délivrées à une fréquence multiple de la fréquence d'oscillation de l'ensemble pendulaire en sortie du circuit d'interfaçage. Dans une forme de réalisation préférentielle, le circuit de gestion d'alimentation comprend en outre une diode anti-retour interposée entre le circuit d'interfaçage et le condensateur tampon, et l'étage d'asservissement MPPT comprend un circuit de détection des périodes de conduction de la diode anti-retour, un circuit extracteur apte à produire un signal représentatif de la valeur courante du rapport cyclique à partir des périodes de conduction et de non-conduction détectées par le circuit de détection, et un circuit de comparaison de la valeur courante du rapport cyclique à une valeur de rapport cyclique optimale prédéterminée. L'étage d'asservissement MPPT peut alors notamment comprendre en outre un circuit de commande, pour : i) si la valeur courante de rapport cyclique est supérieure à la valeur de rapport cyclique optimale, coupler le condensateur tampon au convertisseur régulateur de manière à décharger le condensateur tampon vers une entrée du convertisseur régulateur, ou ii) si la valeur courante de rapport cyclique est inférieure à la valeur de rapport cyclique optimale, découpler le condensateur tampon du convertisseur régulateur de manière à permettre la poursuite de la charge du condensateur tampon par les pulsations successives délivrées par le circuit d'interfaçage.

Le convertisseur régulateur peut notamment être un régulateur abaisseur/élévateur à découpage de type survolteur/dévolteur *buck-boost* sélectivement activable/désactivable par le circuit de commande.

Selon diverses mises en oeuvre avantageuses de la forme de réalisation préférentielle précitée :
- le circuit de détection des périodes de conduction et le circuit extracteur comprennent un comparateur couplé en entrée à la diode anti-retour, apte à détecter la polarité de la différence de potentiel aux bornes de la diode anti-retour, et en aval du comparateur un filtre passe-bas délivrant un signal représentatif de la valeur courante du rapport cyclique ;
- le circuit d'interfaçage comprend un circuit redresseur double alternance, FBR, à pont de diodes ou convertisseur de tension négative NVC (*Negative Voltage Converter*) à MOSFET, et la valeur de rapport cyclique optimale prédéterminée est comprise entre 50% et 55% en termes de rapport cyclique direct, ou entre 45% et 50% en termes de rapport cyclique inverse ;
- le circuit d'interfaçage comprend un circuit FBR à commutation synchronisée de décharge, FBR-SO, et la valeur de rapport cyclique optimale prédéterminée est comprise entre 50% et 52% en termes de rapport cyclique direct, ou entre 48% et 50% en termes de rapport cyclique inverse ;
- le circuit d'interfaçage comprend un circuit FBR à commutation synchronisée d'inductance parallèle de décharge, P-SSHI, et la valeur de rapport cyclique optimale prédéterminée est comprise entre 52% et 60% en termes de rapport cyclique direct, ou entre 40% et 48 % en termes de rapport cyclique inverse ;
- l'étage d'asservissement MPPT comprend : un premier comparateur comparant les tensions aux bornes de la diode anti-retour ; en aval du premier comparateur, un filtre passe-bas délivrant un signal représentatif de la valeur courante du rapport cyclique inverse ; et un second comparateur comparant le signal délivré par le filtre passe-bas à une référence de tension, et délivrant en sortie un signal de pilotage du convertisseur régulateur ;
- l'étage d'asservissement MPPT comprend : un premier comparateur comparant les tensions aux bornes de la diode anti-retour ; en aval du premier comparateur, un premier filtre passe-bas délivrant un premier signal représentatif de la valeur courante du rapport cyclique direct et de la tension délivrée par le circuit d'interfaçage ; un trigger de Schmitt recevant le signal délivré par le premier comparateur ; en aval du trigger de Schmitt, un second filtre passe-bas délivrant un second signal représentatif de la valeur courante du rapport cyclique direct ; et un multiplieur combinant les signaux respectivement délivrés par le premier et le second filtres passe-bas, et délivrant en sortie un signal représentatif d'une estimée de la puissance extraite de la lame piézoélectrique ;
- l'étage d'asservissement MPPT comprend : un premier comparateur comparant les tensions aux bornes de la diode anti-retour ; en aval du premier comparateur, un filtre passe-bas délivrant un premier signal représentatif de la valeur courante du rapport cyclique et du niveau de tension délivré par le circuit d'interfaçage ; en aval du filtre passe-bas, un second comparateur comparant le signal délivré par le filtre passe-bas à une référence de tension, et délivrant en sortie un signal fonction, à un décalage près, de la valeur courante du rapport cyclique direct ; et un multiplieur combinant le signal délivré par le second comparateur et un signal de tension aux bornes du condensateur tampon, et délivrant en sortie un signal représentatif d'une estimée de la puissance extraite de la lame piézoélectrique ;
- dans les deux derniers cas, il peut être en outre prévu un détecteur d'enveloppe recevant en entrée le signal représentatif d'une estimée de la puissance extraite de la lame piézoélectrique, délivré par le multiplieur.

Le module selon l'invention peut notamment être incorporé à un dispositif autonome logeant dans un corps du dispositif : un ensemble électronique ; ledit module de récupération d'énergie ; et un organe de stockage d'énergie pour l'alimentation de l'ensemble électronique. La tension ou le courant continus stabilisés délivrés par le circuit de gestion d'alimentation servent alors à l'alimentation de l'ensemble électronique et/ou à la recharge de l'organe de stockage d'énergie du dispositif autonome.

En particulier, ce dispositif autonome peut être un dispositif médical actif, tel qu'une capsule autonome implantable comprenant un corps de capsule avec un élément d'ancrage à une paroi d'un organe d'un patient. Les sollicitations externes auxquelles.est soumis l'ensemble pendulaire du module de récupération d'énergie sont des sollicitations appliquées au corps de capsule sous l'effet de mouvements de ladite paroi et/ou de variations de débit du flux sanguin dans le milieu environnant.

### DESCRIPTION SOMMAIRE DES DESSINS

On va maintenant décrire un exemple de réalisation de la présente invention en référence aux dessins annexés où les mêmes références désignent d'une figure à l'autre des éléments identiques ou fonctionnellement semblables.
La Figure 1 illustre un dispositif médical de type capsule leadless dans son environnement, implanté au fond du ventricule droit d'un patient.
La Figure 2 présente sous forme schématique les principaux blocs fonctionnels constitutifs de la capsule leadless.
La Figure 3 est un exemple de chronogramme montrant, sur une série de battements cardiaques, les variations instantanées de l'accélération subie par la capsule leadless avec, en regard, les variations correspondantes de la tension en circuit ouvert aux bornes du PZT, produite par les oscillations de l'ensemble pendulaire du PEH, pour une situation correspondant à un état de repos ou de faible activité du patient.
La Figure 4 est homologue de la Figure 3, dans une situation correspondant à un état de stress ou d'effort intense du patient, conduisant à des niveaux d'accélération et de tension beaucoup plus élevés que dans le cas illustré Figure 3.
La Figure 5 est un synoptique d'un circuit PMU à condensateur intermédiaire, circuit auquel sont appliqués les enseignements de l'invention.
La Figure 6 est un exemple de chronogramme des tensions et des courants variables produits par un circuit PMU du type de celui présenté

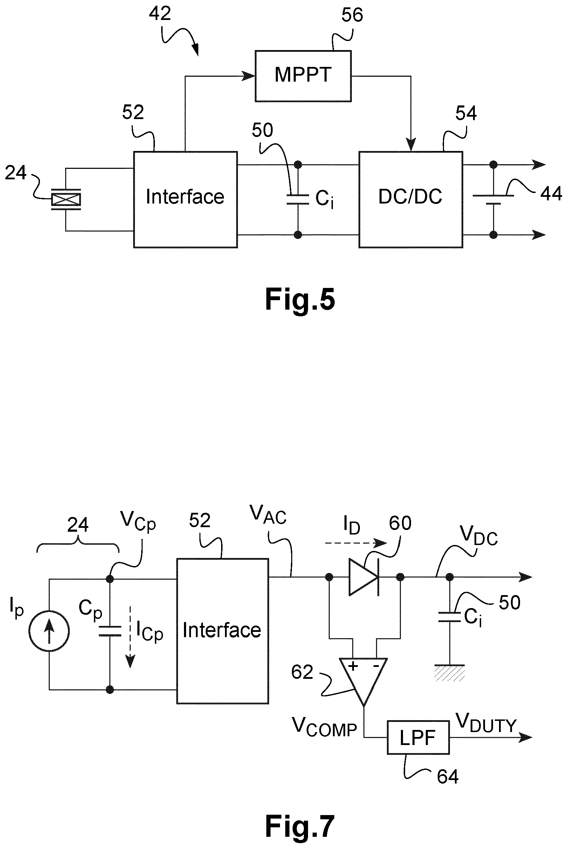

Figure 5, dans une configuration connue en l'absence d'application des enseignements de l'invention.
La Figure 7 est une représentation de principe d'un circuit permettant, selon l'invention, de dériver directement un signal représentatif d'un rapport cyclique de conduction à partir de signaux disponibles au niveau de l'interface avec le PZT.

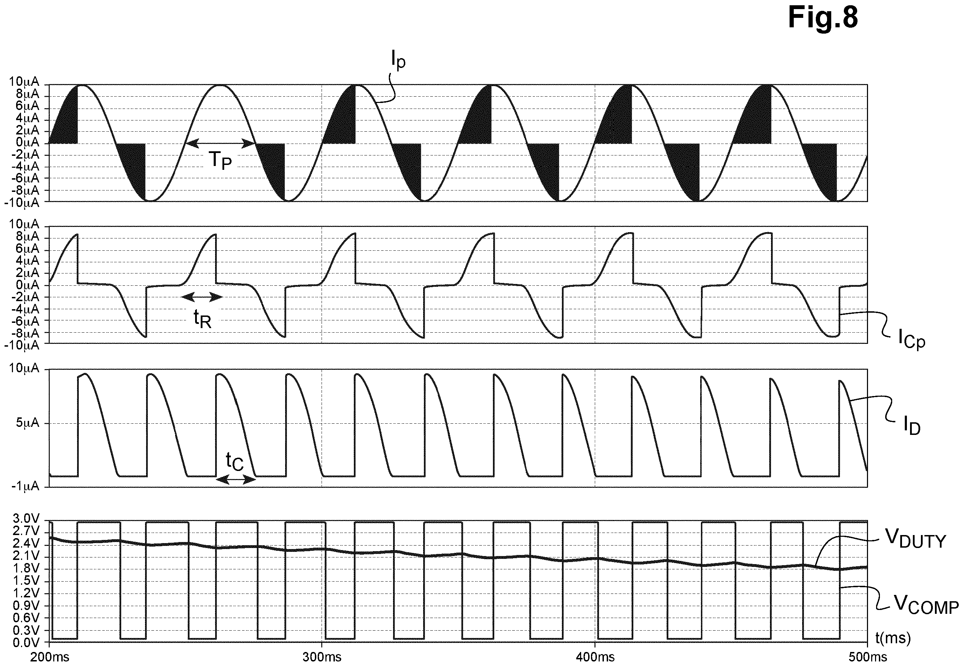

La Figure 8 illustre les variations instantanées des diverses valeurs électriques et signaux du circuit de la Figure 7 dans le cas où le circuit d'interface est un simple redresseur double altèrnance FBR, ainsi que les paramètres définissant le rapport cyclique.

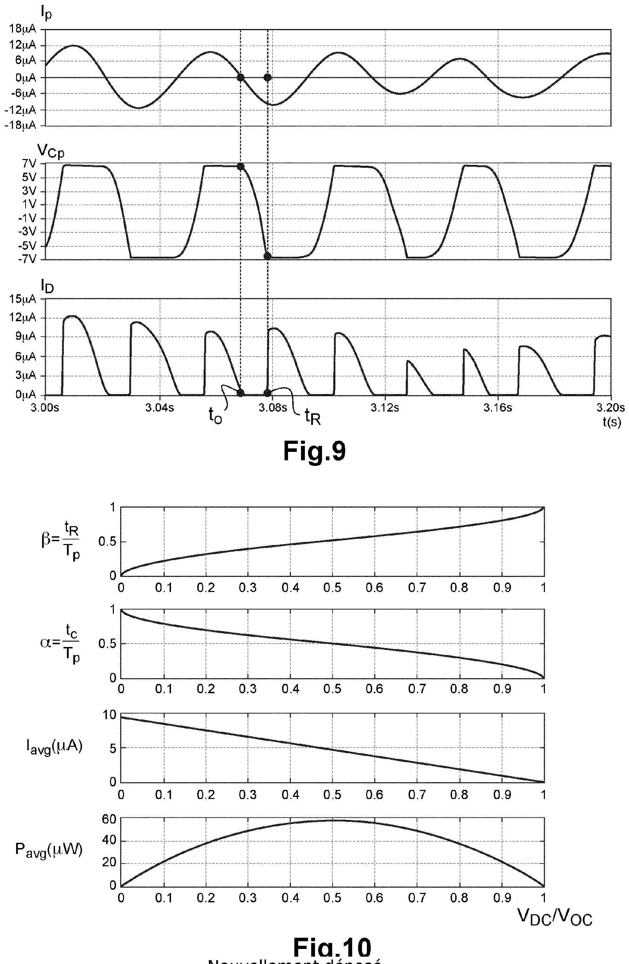

La Figure 9 illustre, dans le cas d'un circuit d'interface constitué d'un simple redresseur double alternance, un chronogramme des variations de divers signaux relevés sur le circuit de la Figure 7 au cours d'oscillations successives du PZT durant un même battement cardiaque.
La Figure 10 illustre les variations des rapports cycliques inverse et direct en fonction du rapport entre la tension en circuit ouvert du PZT et la tension de sortie utile délivrée, ainsi que les variations correspondantes du courant moyen délivré ainsi que de la puissance de sortie extraite.

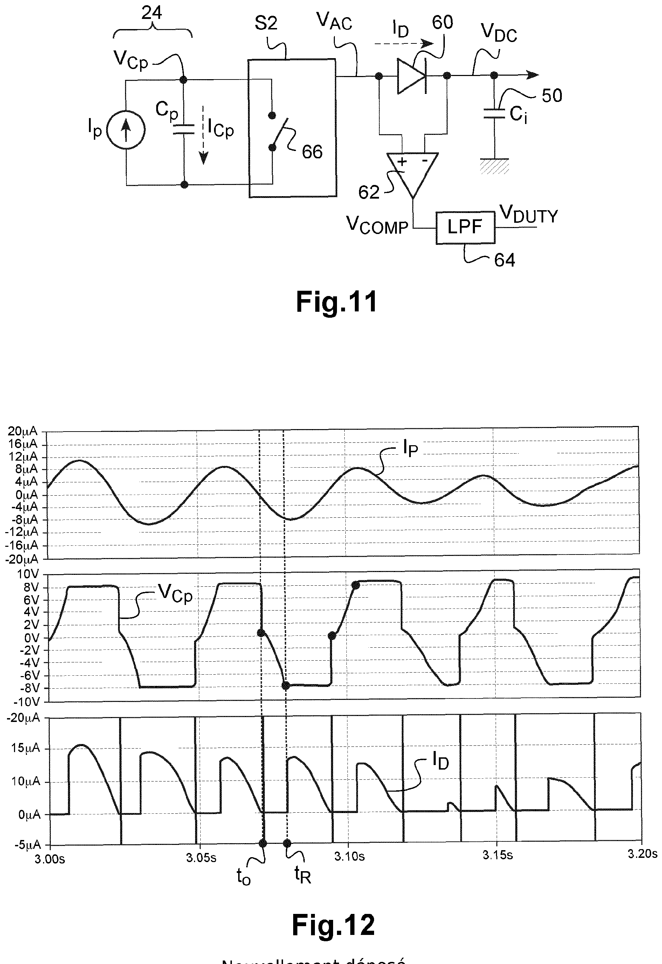

La Figure 11 est homologue de la Figure 7, pour un circuit d'interface de type FBR-SO.
La Figure 12 illustre les variations instantanées des diverses valeurs électriques et signaux du circuit de la Figure 11.

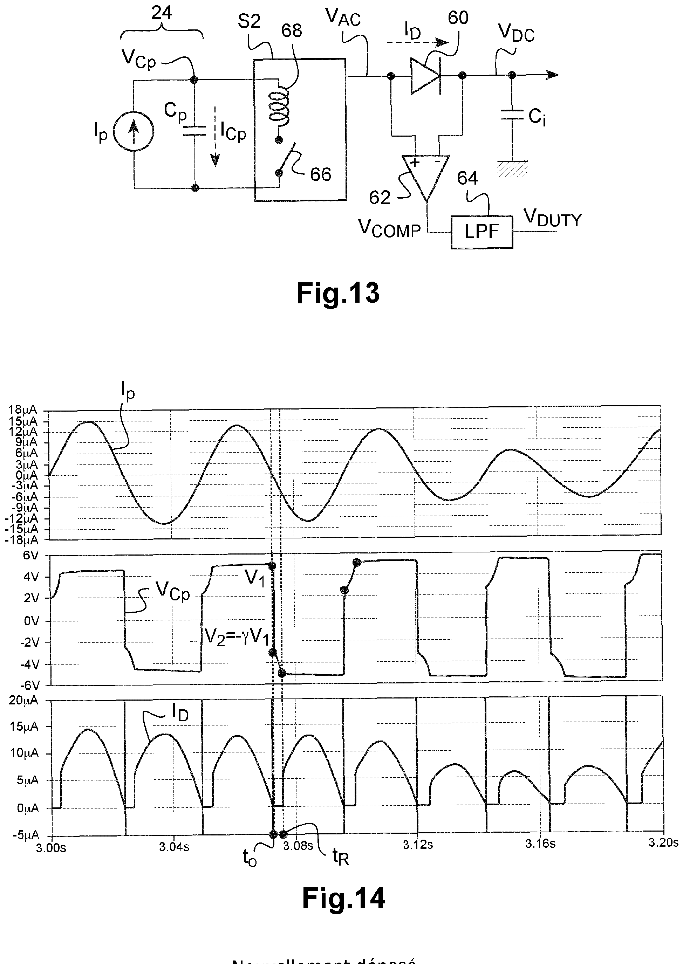

La Figure 13 est homologue de la Figure 7, pour un circuit d'interface de type P-SSHI.
La Figure 14 illustre les variations instantanées des diverses valeurs électriques et signaux du circuit de la Figure 13.

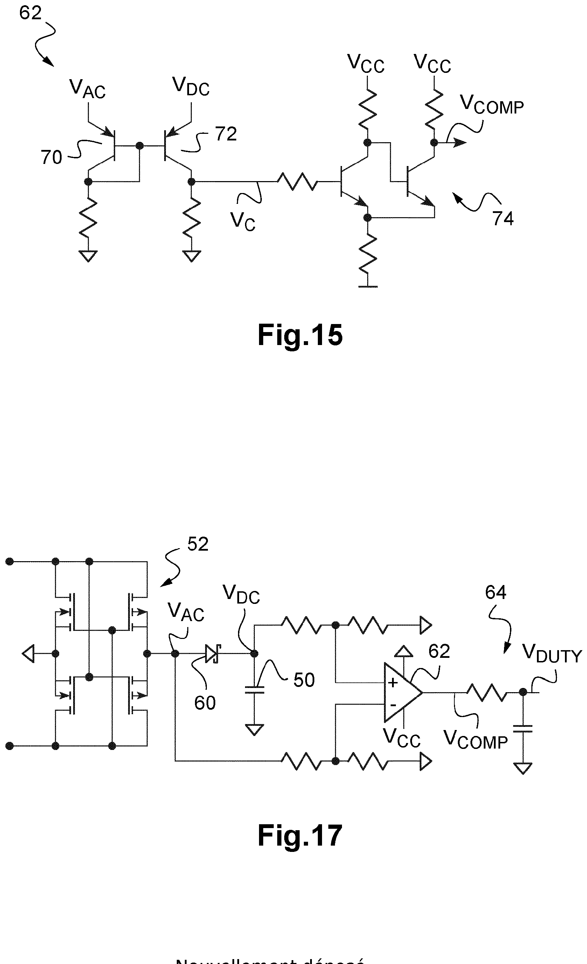

La Figure 15 est un exemple de réalisation de l'étage comparateur du circuit des Figures 7, 11 ou 13.

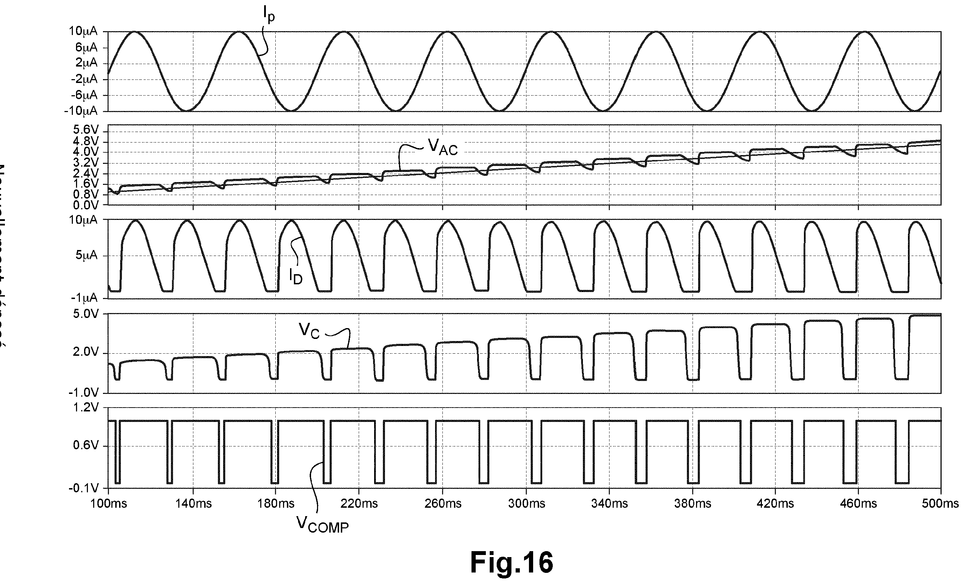

La Figure 16 illustre diverses formes d'onde relevées sur le circuit de la Figure 7, mis en oeuvre avec la structure d'étage comparateur de la Figure 15.
La Figure 17 est un autre exemple de réalisation de l'étage comparateur du circuit des Figures 7, 11 ou 13.

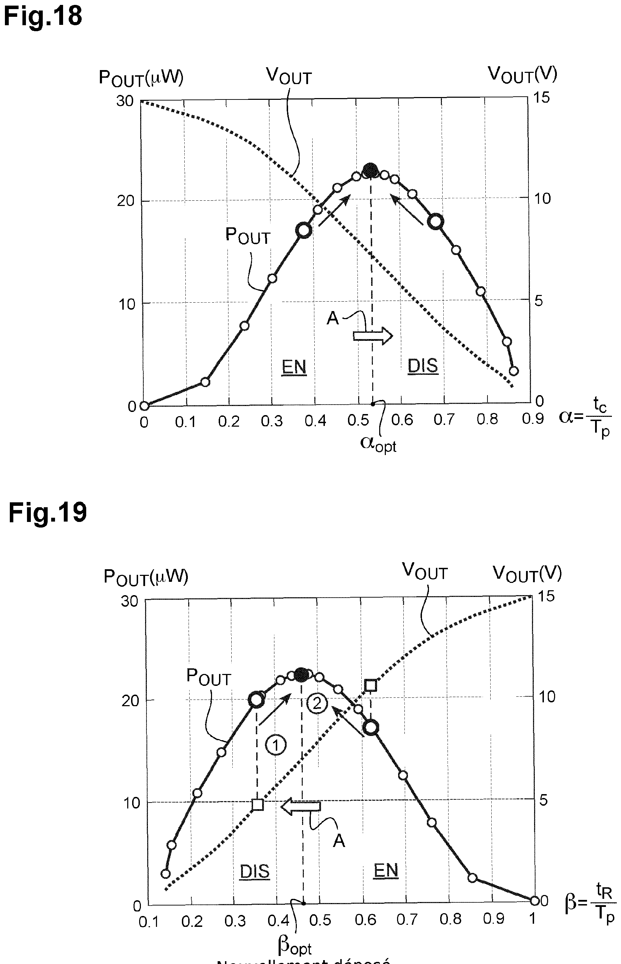

La Figure 18 illustre schématiquement le principe de l'asservissement MPPT mis en oeuvre par la présente invention, montrant les variations respectives, en fonction du rapport cyclique direct déterminé par des circuits tels que ceux des Figures 7, 11 ou 13, de la tension de sortie délivrée par le PEH et de la puissance correspondante extraite du PZT.
La Figure 19 est homologue de la Figure 18, en fonction des variations du rapport cyclique inverse.

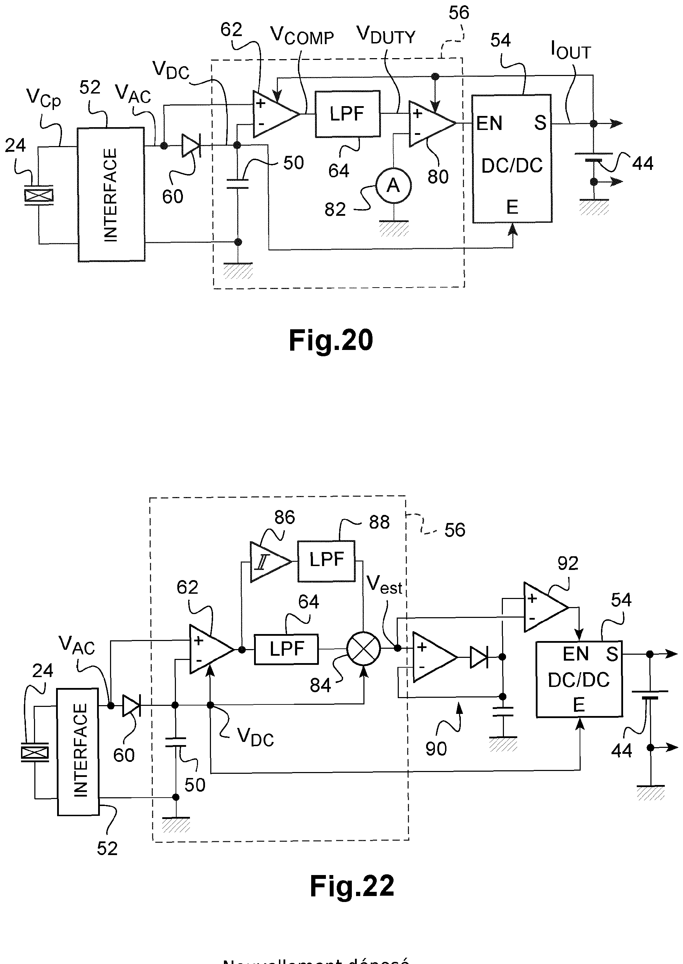

La Figure 20 illustre un premier ensemble de circuits de PEH mettant en oeuvre les enseignements de l'invention, avec une boucle d'asservissement MPPT pilotée par les variations du rapport cyclique inverse.

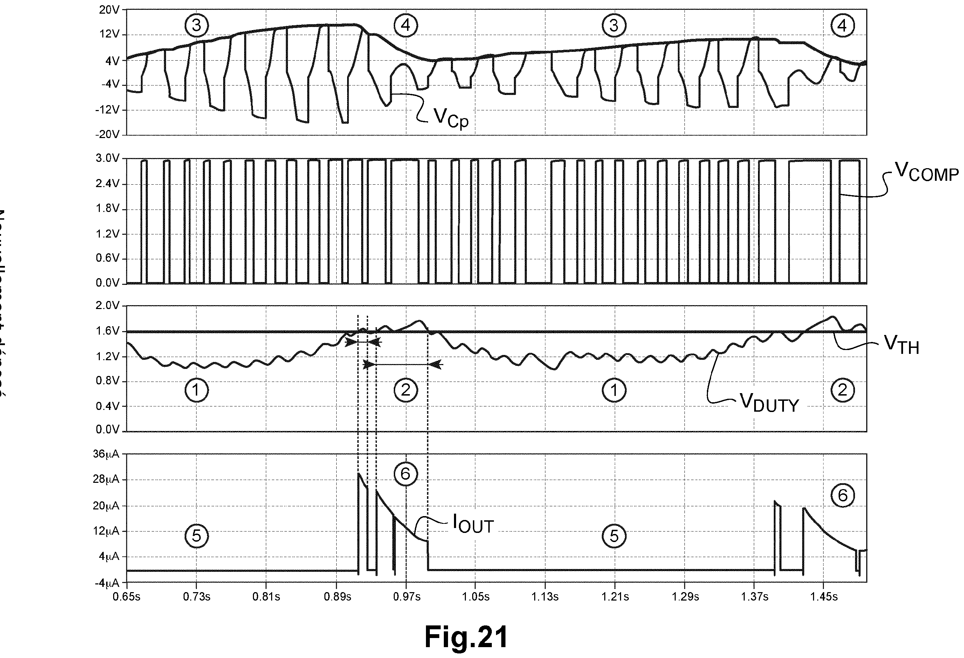

La Figure 21 est une série de chronogrammes montrant les variations instantanées de divers signaux produits par les circuits de la Figure 20 au cours de deux oscillations successives du PZT.
La Figure 22 est homologue de la Figure 20, pour des circuits adaptés à un étage d'alimentation préexistant de type connu comprenant déjà un étage de recherche du maximum de puissance, cet étage étant alors piloté par les variations du rapport cyclique direct.

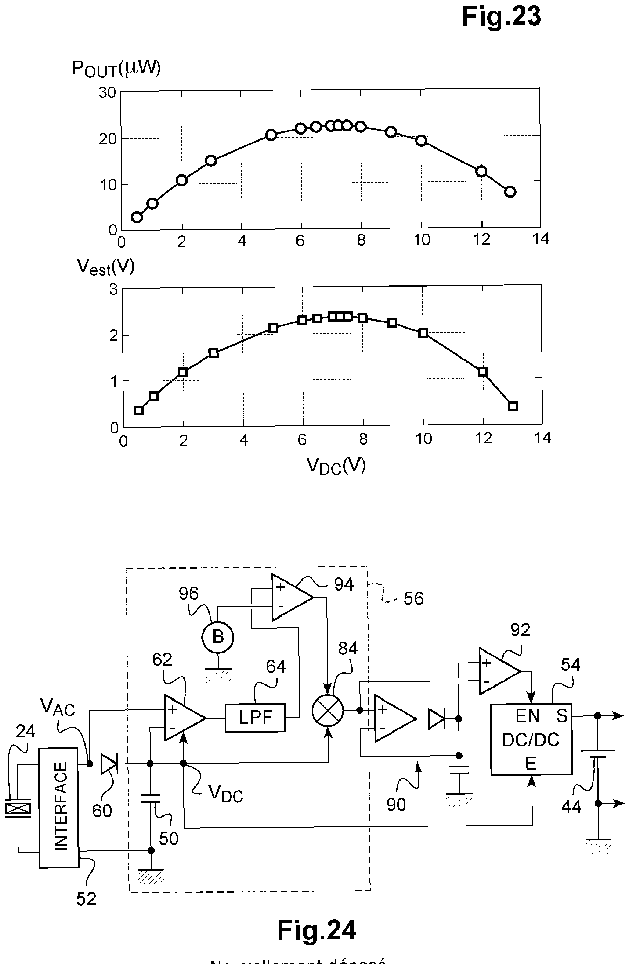

La Figure 23 est une représentation comparative des courbes de puissance estimée et de puissance extraite effectivement mesurée, obtenues avec les circuits de la Figure 22.
La Figure 24 illustre une variante des circuits de PEH de la Figure 22, de fonctionnement plus précis.

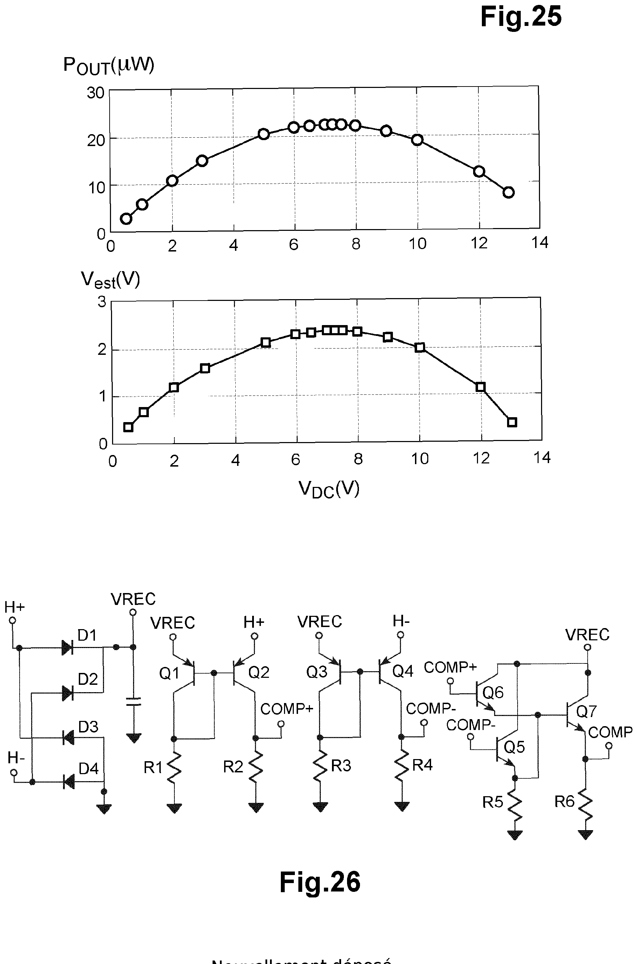

La Figure 25 est homologue de la Figure 23, pour des courbes obtenues avec les circuits de la Figure 24.
La Figure 26 illustre un variante de circuit d'interface à diodes anti-retour et comparateurs.

### DESCRIPTION DÉTAILLÉE

### D'UN MODE DE RÉALISATION PRÉFÉRENTIEL DE L'INVENTION

### Application à un implant cardiaque de type capsule leadless

On va maintenant décrire un exemple de réalisation du dispositif de l'invention, dans une application à une capsule implantable autonome destinée à être implantée dans une cavité cardiaque.

Comme on l'a indiqué plus haut, cette application particulière n'est pas limitative de l'invention, dont les enseignements peuvent être appliqués à de nombreux autre types de dispositifs autonomes incorporant un récupérateur d'énergie de type PEH, que ces dispositifs soient implantables ou non, qu'il s'agisse de dispositifs médicaux ou non.

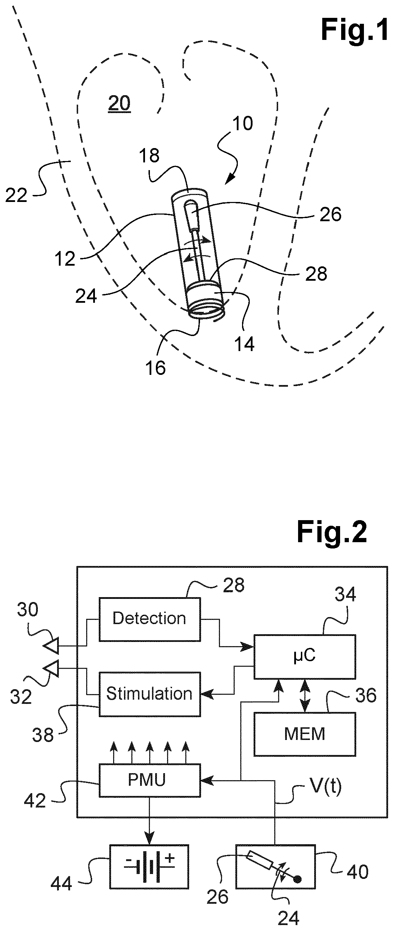

Sur la Figure 1, on a représenté un dispositif de type capsule leadless 10 dans une application à la stimulation cardiaque.

La capsule 10 se présente sous forme extérieure d'un implant avec un corps tubulaire allongé cylindrique 12 enfermant les divers circuits électroniques et d'alimentation de la capsule ainsi qu'un récupérateur d'énergie à ensemble pendulaire. Les dimensions typiques d'une telle capsule sont un diamètre de l'ordre de 6 mm pour une longueur d'environ 15 à 40 mm.

Le corps tubulaire 12 comporte à son extrémité avant (distale) 14 un élément d'ancrage saillant, par exemple une vis hélicoïdale 16 pour maintenir la capsule sur le site d'implantation. D'autres systèmes d'ancrage sont utilisables, et ne modifient en aucune façon la mise en oeuvre de la présente invention. L'extrémité opposée (proximale) 18 de la capsule 10 est une extrémité libre, qui est seulement pourvue de moyens (non représentés) de liaison temporaire à un cathéter-guide ou autre accessoire d'implantation utilisé pour la mise en place ou l'explantation de la capsule, qui est ensuite désolidarisé de cette dernière.

Dans l'exemple illustré Figure 1, la capsule leadless 10 est un implant endocavitaire implanté à l'intérieur d'une cavité 20 du myocarde 22, par exemple à l'apex du ventricule droit. En variante, toujours dans une application à la stimulation cardiaque, la capsule peut être également implantée sur le septum interventriculaire ou sur une paroi auriculaire, ou encore être une capsule épicardique placée sur une région externe du myocarde, ces différents modes d'implantation ne modifiant en aucune façon la mise en oeuvre de la présente invention. Pour assurer les fonctions de détection/stimulation, une électrode (non représentée) en contact avec le tissu cardiaque au site d'implantation assure le recueil des potentiels de dépolarisation cardiaque et/ou l'application d'impulsions de stimulation. Dans certaines formes de réalisation, la fonction de cette électrode peut être assurée par la vis d'ancrage 16, qui est alors une vis active, électriquement conductrice et reliée au circuit de détection/ stimulation de la capsule.

La capsule leadless 10 est par ailleurs dotée d'un module récupérateur d'énergie dit "PEH", comprenant un ensemble pendulaire inertiel qui oscille à l'intérieur de la capsule au gré des diverses sollicitations externes auxquelles la capsule est soumise. Ces sollicitations peuvent notamment résulter : des mouvements de la paroi à laquelle est ancrée la capsule, qui sont transmis au corps tubulaire 12 par la vis d'ancrage 16 ; et/ou des variations du débit du flux sanguin dans le milieu environnant la capsule, qui produisent des oscillations du corps tubulaire 12 au rythme des battements cardiaques ; et/ou des vibrations diverses transmises par les tissus cardiaques.

L'ensemble pendulaire est constitué d'une lame piézoélectrique 24 encastrée en 28 à l'une de ses extrémités et dont l'extrémité opposée, libre, est couplée à une masse inertielle mobile 26. La lame piézoélectrique 24 est une lame flexible élastiquement déformable qui constitue avec la masse inertielle 26 un système pendulaire de type masse-ressort. Du fait de son inertie, la masse 26 soumet la lame 24 à une déformation de type vibratoire de part et d'autre d'une position neutre ou non déformée correspondant à une position stable de repos en l'absence de toute sollicitation.

De fait, pour ce qui est de son comportement mécanique, cet ensemble peut être assimilé à une structure de type "poutre encastrée-libre" (*fixed-free beam*), présentant une fréquence d'oscillation propre qui est ici la fréquence sur laquelle oscille le système masse-ressort. On notera que cette fréquence d'oscillation propre, typiquement de l'ordre de quelques dizaines de hertz, est notablement supérieure à la fréquence des sollicitations cycliques externes qui correspondant à la fréquence des battements cardiaques (quelques hertz tout au plus). Ainsi, à chaque contraction cardiaque la masse inertielle (ou autre organe mécanique fonctionnellement analogue) sera sollicitée avec une amplitude plus ou moins importante, puis le système pendulaire oscillera plusieurs fois avec des amplitudes décroissantes (rebonds caractéristiques d'une oscillation périodique amortie), et enfin se stabilisera jusqu'au battement cardiaque suivant, où le cycle sollicitation/oscillations se reproduira de façon comparable.

La lame 24 assure en outre une fonction mécanoélectrique de transducteur piézoélectrique (PZT) permettant de convertir en charges électriques la contrainte mécanique de flexion qui lui est appliquée. Ces charges sont collectées par des électrodes à la surface de la lame de manière à produire un signal électrique qui, après redressement, stabilisation et filtrage, alimentera les circuits électroniques de la capsule. La Figure 2 est un synoptique des divers circuits électriques et électroniques intégrés à la capsule leadless, présenté sous forme de blocs fonctionnels.

Le bloc 28 désigne un circuit de détection de l'onde de dépolarisation cardiaque, qui est relié à une électrode de cathode 30 en contact avec le tissu cardiaque et à une électrode d'anode 32 associée, par exemple une électrode annulaire formée sur le corps tubulaire de la capsule. Le bloc de détection 28 comprend des filtres et des moyens de traitement analogique et/ou numérique du signal recueilli. Le signal ainsi traité est appliqué à l'entrée d'un microcalculateur 34 associé à une mémoire 36. L'ensemble électronique comporte également un circuit de stimulation 38 opérant sous le contrôle du microcalculateur 34 pour délivrer en tant que de besoin au système d'électrodes 30, 32 des impulsions de stimulation du myocarde.

Il est par ailleurs prévu un circuit récupérateur d'énergie ou PEH 40, constitué par l'ensemble pendulaire formé par la lame piézoélectrique 24 et la masse inertielle 26 décrits plus haut en référence à la Figure 1. Comme la lame piézoélectrique 24 assure aussi une fonction de transducteur mécano-électrique, elle convertit en charges électriques les sollicitations mécaniques subies et produit un signal électrique variable V(t), qui est un signal alternatif qui oscille i) à la fréquence d'oscillation libre de l'ensemble pendulaire lame 24/masse 30, et ii) au rythme des battements successifs du myocarde auquel la capsule est couplée.

Le signal électrique variable V(t) est délivré à un circuit de gestion de l'énergie ou PMU 42. Le PMU 42 redresse et régule le signal V(t) de manière à produire en sortie une tension ou un courant continus stabilisés servant à l'alimentation des divers circuits électroniques et à la recharge d'une microbatterie intégrée 44.

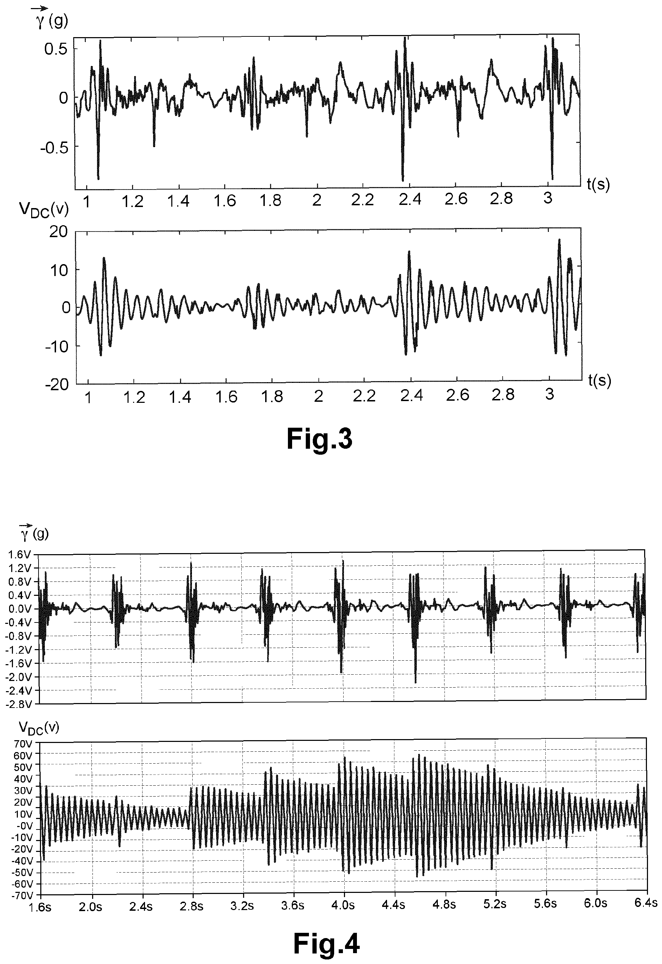

La Figure 3 est un exemple de chronogramme montrant, sur une série de battements cardiaques, les variations instantanées de l'accélération subie par la capsule leadless avec, en regard, les variations correspondantes de la tension en circuit ouvert Voc aux bornes du PZT, produites par les oscillations de l'ensemble pendulaire du PEH, pour une situation correspondant à un état de repos ou de faible activité du patient.

Cette tension Voc se présente sous forme d'un signal récurrent se répétant au rythme des battements cardiaques successifs, avec une suite d'oscillations sinusoïdales amorties comprenant un premier pic de forte amplitude suivi d'une série de "rebonds" d'amplitudes décroissantes, qui se prolongent jusqu'à une nouvelle contraction du myocarde produisant des variations similaires de la tension. L'ordre de grandeur de la fréquence de la récurrence des cycles cardiaques est typiquement de 1 à 2 Hz (60 à 120 bpm). La fréquence propre de l'ensemble pendulaire, quant à elle, est déterminée par la géométrie de la lame 24 (principalement sa longueur et son épaisseur), par l'élasticité du matériau qui la compose, et par la masse de la masse inertielle 28. Cette fréquence propre d'oscillations libres présente une valeur beaucoup plus élevée que la fréquence du rythme cardiaque, typiquement une fréquence de l'ordre de 15 à 40 Hz.

Dans cette situation, pour des accélérations modérées (typiquement d'une fraction de g), la tension en circuit ouvert Voc, c'est-à-dire en l'absence de charge connectée aux bornes du PZT, varie dans une plage de l'ordre de ± 12 V dans l'exemple illustré.

En revanche, comme illustré Figure 4, dans une situation correspondant à un état de stress ou d'effort intense du patient, au lieu d'une position statique ou d'un effort modéré. Les accélérations peuvent alors atteindre 2 g, conduisant à une tension V_{OC} pouvant varier de ± 5 V à ± 60 V crête-à-crête.

### Principe général d'un récupérateur d'énergie à asservissement MPPT

La Figure 5 est un synoptique d'un circuit récupérateur de type PEH à condensateur intermédiaire, selon une configuration d'ensemble en elle-même connue, par exemple d'après l'article de Ottman et les US 8 026 650 B2 et US 2005/285728 A1 cités en introduction.

Cet agencement comprend un condensateur intermédiaire de lissage 50, de capacité Ci, chargé par un circuit d'interface 52 interposé entre le PZT 24 et le condensateur intermédiaire 50.

Le circuit d'interface 52 recueille le signal électrique alternatif oscillant amorti produit par le PZT 24 et délivre en sortie une tension redressée permettant de charger le condensateur intermédiaire 50. Dans sa forme la plus simple, le circuit d'interface 52 est un redresseur double alternance FBR à pont de diodes, mais d'autres circuits plus complexes peuvent être utilisés pour optimiser l'extraction de l'énergie produite par le PZT, notamment un circuit d'interface de type SSHI.

Le condensateur intermédiaire 50 permet de lisser les variations de la tension redressée, qui est appliquée en entrée d'un convertisseur continu-continu (DC/DC) 54, typiquement de type *buck-boost* (dévolteur et/ou survolteur) de manière à délivrer en sortie une tension stabilisée à un niveau permettant la recharge de la batterie 44 et l'alimentation des circuits situés en aval de cette batterie.

Pour optimiser l'extraction de puissance, un étage d'asservissement 56 de type MPPT (*Maximum Power-Point Tracking*) pilote le convertisseur *buck-boost* 54 de manière à maximiser le transfert vers la batterie 44 de l'énergie accumulée sur le condensateur 50.

Toutefois, les asservissements MPPT conventionnels, même s'ils sont adaptés au recueil d'énergie par un PEH, ne permettent pas de répondre aux multiples contraintes évoquées plus haut, propres notamment aux implants cardiaques miniaturisés tels que les capsules leadless.

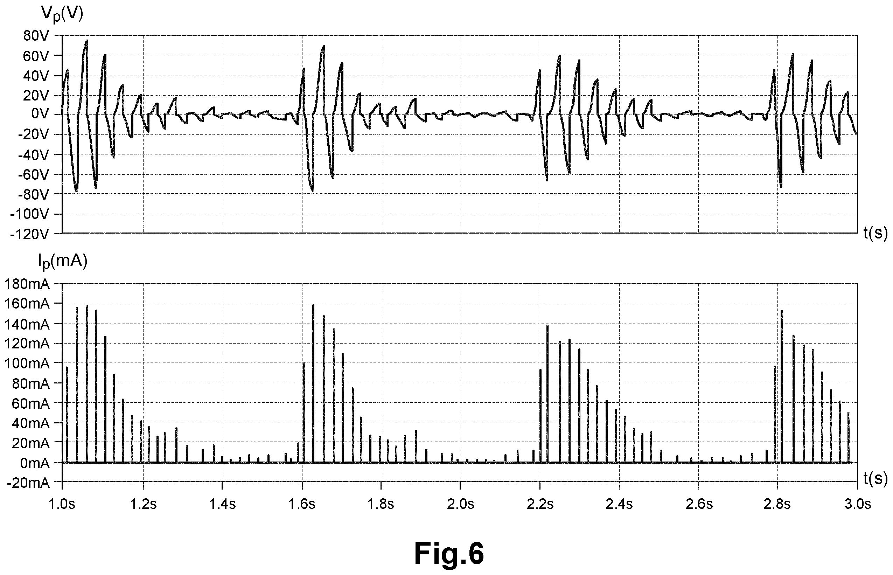

*En premier lieu*, l'utilisation de circuits d'interface 52 de type conventionnel S-SSHI ou SECE, incorporant une inductance pour transférer à la batterie la tension produite par le PZT, n'est pas compatible avec la recharge des microbatteries telles que celles utilisées dans les implants leadless, dont la faible capacité limite le courant maximal de recharge à une valeur de l'ordre de 5 mA. En effet, si l'on se réfère à la Figure 6 qui est un exemple de chronogramme des tensions et des courants variables produits par un circuit PMU du type de celui présenté Figure 5, dans une configuration connue en l'absence d'application des enseignements de l'invention, on constate que la tension aux bornes du PZT peut atteindre jusqu'à ± 60 V, ce qui conduit, au moment de la commutation de l'inductance, à un courant de sortie extrêmement élevé, pouvant atteindre typiquement 160 mA pour une inductance de 1 mH.

*En second lieu*, avec un signal tel que celui produit par un PZT incorporé à une capsule leadless, la périodicité des battements cardiaques (1 à 2 Hz) est très éloignée de la fréquence harmonique du PZT (de l'ordre de 15 à 40 Hz), ce qui ne permet pas une optimisation précise et instantanée du transfert d'énergie par l'asservissement MPPT.

*En troisième lieu*, les asservissements MPPT connus fonctionnent selon des techniques mettant en oeuvre des algorithmes relativement lents et consommateurs d'énergie.

Ces algorithmes sont habituellement du type "P&O" (*Perturbate-and-Observe*), consistant à provoquer un décalage de la tension de sortie et de mesurer la variation de puissance observée pour recaler la tension de sortie vers un niveau maximisant cette puissance, ou de type "FOC" (*Fractional Open-Circuit*), consistant à déconnecter temporairement le convertisseur du condensateur intermédiaire, puis mesurer la tension aux bornes de ce dernier en circuit ouvert et recaler la tension sur une valeur correspondant à la moitié de cette tension en circuit ouvert, valeur supposée correspondre au maximum de puissance délivré en sortie. Dans l'un ou l'autre cas, pour trouver le sens dans lequel doit être modifiée la tension de sortie, l'algorithme provoque une perte temporaire de puissance du système en forçant ce dernier à fonctionner hors de sa zone optimale. Cette manière de procéder est difficilement envisageable dans le cas d'un implant cardiaque où, idéalement, l'ajustement du transfert de puissance doit être fait à chaque cycle cardiaque, et en minimisant les pertes d'énergie propres à - l'asservissement, au risque de ne pas suffisamment charger la microbatterie.

En effet, au cours d'un même cycle cardiaque la tension en circuit ouvert Voc du PZT est susceptible de varier d'environ 5 à 24 volts, correspondant à une valeur optimale Voc de 2,5 à 12 volts pour un optimum fixé à Voc/2. Un algorithme d'asservissement MPPT qui fonctionnerait sur la base de la mesure de la tension en circuit ouvert Voc devrait effectuer plusieurs mesures de Voc au cours d'un même cycle cardiaque pour suivre efficacement le point de fonctionnement optimal de puissance délivrée. En effet, la puissance délivrée peut varier au cours d'un même cycle cardiaque entre environ 2 µW et 50 µW, de sorte que le point de consigne de l'asservissement varie en permanence, imposant un nombre de mesures très élevé pour obtenir un asservissement réellement performant.

### Principe de l'invention et extraction de l'information de rapport cyclique

La présente invention propose une autre technique pour maximiser l'extraction de l'énergie produite par le PZT, que l'on va maintenant décrire en référence aux Figures 7 à 21.

Le principe de l'invention consiste (i) à dériver directement un signal représentatif d'un rapport cyclique de conduction à partir de signaux directement disponibles au niveau de l'interface avec le PZT, et (ii) à piloter de façon dynamique l'asservissement MPPT à partir de cette valeur de rapport cyclique.

La Figure 7 décrit de façon schématique la manière de détecter les périodes de conduction et de non-conduction d'une diode montée en sortie d'un circuit d'interface de PMU tel que celui de la Figure 5, et d'en dériver une valeur courante instantanée de rapport cyclique.

L'interface 52 délivre une tension V_{AC}, qui est par exemple une tension simplement redressée par un circuit double alternance FBR. Cette tension V_{AC} est appliquée à l'anode d'une diode anti-retour 60 dont la cathode est reliée au condensateur intermédiaire 50, en appliquant ainsi à ce dernier une tension de charge lorsque la diode 60 est passante. La tension aux bornes du condensateur 50, redressée et filtrée par la capacité Ci de ce condensateur 50, est une tension V_{DC} filtrée, sensiblement continue.

Le courant I_{D} traversant la diode produit entre les bornes de cette dernière une chute de tension égale à la tension directe de la diode. La différence de tension correspondante est détectée par un comparateur 62 dont les entrées sont reliées aux bornes respectives de la diode 60. La sortie V_{COMP} du comparateur 62 est appliquée à un filtre passe-bas LPF 64 délivrant en sortie un signal V_{DUTY}.

La Figure 8 illustre une série de chronogrammes relevés en différents points du circuit de la Figure 7 au cours de six cycles successifs de vibration du PZT, relevés au cours d'un même cycle de sollicitation mécanique du PEH, notamment au cours d'un même battement cardiaque de l'implant porté par le patient.

Les oscillations du PZT produisent, par effet piézoélectrique, un courant I_{P} (premier chronogramme de la Figure 8) de forme approximativement sinusoïdale et de demi-période T_{P}. Au début de la variation, le courant I_{P} charge la capacité interne Cₚ du PZT (représentée sur le schéma équivalent de la Figure 7), jusqu'à une tension V_{AC}. Le courant de charge I_{Cp} (deuxième chronogramme de la Figure 8) généré à l'intérieur du PZT et chargeant la capacité interne Cₚ croît pendant une durée t_{R} de temps mort (zone sombre sur la sinusoïde du premier chronogramme) pendant laquelle la tension V_{AC} est insuffisante pour mettre en conduction la diode 60 (V_{AC} < V_{DC}).

Lorsque V_{AC} atteint le niveau de la tension V_{DC} présente aux bornes du condensateur intermédiaire 50, la diode 60 entre en conduction et est parcourue par un courant I_{D} (troisième chronogramme de la Figure 8), cette conduction se poursuivant pendant une durée te jusqu'au passage par zéro suivant du courant I_{D}.

Comme on peut le voir sur la figure, la durée de conduction te est relativement longue pour les premiers cycles d'oscillation du PZT, puis décroît progressivement au cours des cycles suivants, et continuera à décroître jusqu'au prochain battement cardiaque qui sollicitera à nouveau mécaniquement le PZT.

De façon corrélative, le comparateur 62 produit en sortie un signal V_{COMP} (quatrième chronogramme de la Figure 8) se présentant sous forme d'impulsions alternant entre un niveau haut correspondant à un état de conduction de la diode 60 (V_{AC} > V_{DC}), et un niveau bas correspondant à un état bloquant de la diode 60 (V_{AC} < V_{DC}).

La durée des impulsions au niveau haut (état de conduction de la diode) diminue progressivement au fil des cycles d'oscillation successifs du PZT, et la valeur filtrée V_{DUTY} du signal impulsionnel V_{COMP} donne une représentation continue, non impulsionnelle, de cette décroissance progressive.

Plus précisément, la valeur moyenne du signal de sortie filtré V_{DUTY} est représentative d'une mesure du rapport cyclique α = t_{C}/T_{P}, défini comme le ratio entre la période te pendant laquelle la diode est en conduction et la demi-période d'oscillation propre T_{P} du PEH. De la même façon, le rapport cyclique inverse β = t_{R}/T_{P} est défini comme le ratio entre la période t_{R} pendant laquelle la diode est bloquée et la demi-période d'oscillation propre T_{P} du PEH. La mesure du rapport cyclique inverse β est réalisable en inversant les deux entrées du comparateur.

### Utilisation de l'information de rapport cyclique

Ces paramètres α ou β seront utilisés par la suite pour piloter l'asservissement MPPT du PEH, dont le point de fonctionnement sera optimisé dynamiquement pour maximiser la puissance extraite du PZT.

La Figure 9 illustre respectivement, sur une série de courbes relevées dans une configuration où le circuit d'interface 52 est un simple redresseur double alternance FBR à pont de diodes :
le courant I_{P} traversant le PZT au cours de cycles d'oscillation successifs ;
la tension de sortie V_{Cp} correspondante aux bornes de la capacité interne Cₚ du PZT ; et
le courant I_{D} traversant la diode 60, qui est le courant délivré au condensateur intermédiaire 50 qui servira pour recharger la microbatterie 44 et alimenter les divers circuits électroniques et capteurs de l'implant.

La tension de sortie maximale V_{DC} qu'il est possible d'obtenir est égale à la tension maximale en circuit ouvert Voc aux bornes du PZT, diminuée des chutes de tension V_{D} aux bornes des diodes en série entre le PZT et le condensateur intermédiaire 50, incluant les diodes du redresseur double alternance de l'interface 52 (à savoir les deux diodes du pont de diodes qui se trouvent en conduction au cours d'une alternance donnée). Après le passage par zéro du courant I_{P} délivré par le PZT (à l'instant t₀), la diode 60 n'entre pas en conduction avant un temps t_{R} correspondant au temps nécessaire pour que la tension sur la capacité interne Cₚ du PZT bascule de (V_{DC} + 2V_{D}) à - (V_{DC} + 2 Vo). Lorsque la tension V_{DC} sur le condensateur intermédiaire 50 augmente, cette durée t_{R} augmente, et le temps de conduction tc diminue corrélativement.

De fait, en début de cycle pour les premières oscillations du PZT après un battement cardiaque, le temps de conduction est relativement long mais en revanche la tension Voc accumulée sur le condensateur intermédiaire 50 est faible, tandis qu'en fin de cycle cardiaque le temps de conduction est relativement court mais en revanche la tension V_{DC} accumulée sur le condensateur intermédiaire 50 est beaucoup plus élevée.

L'optimum d'extraction de l'énergie ne se situe donc pas dans ces états extrêmes, mais dans un état intermédiaire, que l'on cherche à déterminer pour définir un point de consigne de l'asservissement MPPT.

La Figure 10 présente diverses courbes permettant notamment d'illustrer la relation existante entre les variations du rapport cyclique direct α ou inverse β et la puissance moyenne extraite P_{avg}. Ces variations sont données en fonction du rapport V_{DC}/V_{OC} entre la tension utile V_{DC} disponible en sortie pour la recharge du condensateur intermédiaire 50 et la tension maximale en circuit ouvert Voc que peut délivrer le PZT entre ses bornes. On démontre que, si l'on néglige la chute de tension aux bornes de la diode 60, la puissance extraite du PZT passe par un maximum pour des valeurs de rapport cyclique α = 0,5 (ou β = 0,5), c'est-à-dire lorsque la tension de sortie V_{DC} est égale à la moitié de la tension maximale en circuit ouvert Voc aux bornes du PZT.

Si l'on prend en compte les chutes de tension des diodes du circuit, ainsi que l'offset et l'hystérésis introduits par le circuit comparateur 62, on trouve que l'optimum se situe en réalité à une valeur un peu inférieure à 0,5 Voc, correspondant à un rapport cyclique direct α un peu supérieur à 50 % (ou un rapport cyclique inverse β un peu inférieur à 50 %). Concrètement, l'optimum peut être situé autour de α = 53 %, soit dans une plage α = 50-55 % (ou autour de β = 47 %, soit une plage β = 45-50 %).

Ces valeurs d'optimum correspondent au cas, indiqué plus haut, d'une interface 52 mettant en oeuvre un simple redresseur double alternance FBR à pont de diodes.

Elles sont à adapter, théoriquement et expérimentalement, si d'autres schémas de circuit d'interface sont utilisés.

Ainsi, comme illustré Figure 11, l'interface peut être un circuit de type "FBR-SO" (*FBR-Switch Only*), c'est-à-dire un circuit FBR complété par un commutateur de court-circuit 66 monté en parallèle sur le PZT et qui se ferme au moment du passage par zéro du courant afin de décharger immédiatement la capacité interne du PZT. Les variations correspondantes de I_{P}, V_{Cp} et I_{D} sont illustrées sur la Figure 12.

Du fait de la fermeture du commutateur 66, la tension Voc en circuit ouvert est doublée par rapport à un circuit FBR simple, ce qui modifie le temps t_{R} avant que la diode n'entre en conduction. Si l'on prend en compte les caractéristiques des diodes utilisées et le retard introduit par la détection du passage par zéro (qui requiert un circuit supplémentaire), le rapport cyclique optimal est alors en pratique situé dans une plage α = 50-52 % (soit β = 48-50 %).

Un autre circuit d'interface utilisable est un circuit de type P-SSHI évoqué plus haut, qui est essentiellement, comme illustré Figure 13, un circuit de type FBR-SO dans lequel une inductance 68 a été ajoutée en série avec le commutateur 66 de court-circuit monté aux bornes du PZT. Les variations correspondantes de I_{P}, V_{Cp} et I_{D} sont illustrées sur la Figure 14. En pratique, avec un tel circuit d'interface P-SSHI le rapport cyclique optimal se situe dans une plage α = 52-60 % (soit β = 40-48 %).

Ainsi, comme on peut le voir, bien que le rapport cyclique optimal théorique soit dans tous les cas de 50 %, compte tenu des diverses considérations pratiques liées à la configuration choisie pour le circuit d'interface et aux composants matériels mis en oeuvre, le rapport cyclique optimal réel, bien que situé autour de 50 %, peut se situer dans une plage voisine, mais différente, de cette valeur théorique.

### Exemples de circuits permettant d'extraire un signal de rapport cyclique

La Figure 15 est un exemple de réalisation de l'étage comparateur 62 du circuit des Figures 7, 11 ou 13.

Ce comparateur est réalisé sous forme d'un circuit autoalimenté avec deux transistors bipolaires 70, 72 montés en opposition et recevant sur leurs émetteurs respectifs les signaux V_{AC} et V_{DC} recueillis aux bornes de la diode 60. Pour obtenir un signal binaire V_{COMP} représentatif du rapport cyclique de conduction de la diode 60, un étage trigger de Schmitt 74 est couplé à la sortie du comparateur 70, 72 de manière à provoquer une saturation ou "clamping" du signal Vc.

La Figure 16 illustre sur cinq chronogrammes les variations respectives concomitantes (i) du courant I_{P} délivré par le PZT, (ii) de la tension V_{AC} en sortie du circuit d'interface 52, (iii) du courant I_{D} traversant la diode 60, (iv) du signal Vc en sortie de la paire de transistors 70, 72, et (v) du signal de rapport cyclique V_{COMP} délivré en sortie du trigger de Schmitt 74. On constate qu'au fil des cycles successifs d'oscillation de la lame, l'amplitude maximale du signal Vc augmente progressivement, de façon proportionnelle à la valeur moyenne de la tension V_{AC}, le trigger de Schmitt 74délivrant un signal binaire V_{COMP} représentatif du rapport cyclique de conduction de la diode 60.

La Figure 17 illustre un autre exemple de réalisation pratique de l'étage comparateur des Figures 7, 11 ou 13.

Cet exemple met en oeuvre un unique composant comparateur 62, utilisant une alimentation externe Vcc. Ce comparateur peut être incorporé à un circuit ASIC en technologie CMOS, ou réalisé à partir de composants discrets de type connu couramment disponibles COTS (*Commercial Off-The-Shelf*) à transistors PMOS/NMOS, qui présentent par rapport à des transistors bipolaires l'avantage de ne pas opposer de tension de seuil de diode, donc sans zone morte ni perte de charge significative. En entrée du comparateur 62, des diviseurs de tension limitent les niveaux de tension appliqués aux entrées du circuit comparateur à des niveaux ne dépassant pas la tension d'alimentation Vcc.

Sur cette même Figure 17 a été également illustrée une autre variante, utilisable indépendamment du type d'étage comparateur utilisé, dans laquelle, pour obtenir la tension redressée positive V_{AC} à partir du signal délivré par le PZT, l'étage d'interface 52 est réalisé sous forme d'un convertisseur de tension négative NVC (*Negative Voltage Converter*) en lieu et place d'un pont de diodes. Le NVC est réalisé à base de MOSFET, évitant les inconvénients d'un pont de diodes, notamment la chute de tension directe des diodes lorsqu'elles sont conductrices, conduisant donc à un meilleur rendement du circuit d'interface.

Le montage NVC à base de MOSFET n'inclut pas de diode anti-retour. C'est pourquoi il est suivi par une diode passive ou active pour empêcher un retour de courant de la capacité Ci vers le PZT.

La Figure 26 présente une autre variante dans laquelle le circuit d'interface 52 est un redresseur à quatre diodes avec deux diodes anti-retour D1 et D2. Le comparateur qui sert à estimer le rapport cycle est constitué de deux comparateurs avec les sorties COMP+ et COMP-. Un circuit OU logique permet de combiner les sorties des deux comparateurs pour former un signal COMP représentatif de la conduction des deux diodes D1 ou D2, utilisable pour estimer le rapport cyclique.

### Exemples de boucle MPPT asservie sur le rapport cyclique

On va maintenant décrire en référence aux Figures 18 à 25 le principe de l'asservissement MPPT mis en oeuvre, selon les enseignements de la présente invention, à partir du rapport cyclique, direct ou inverse, déterminé par des circuits tels que ceux des Figures 7, 11 ou 13 décrits plus haut.

Sur la Figure 18 on a illustré schématiquement le principe d'un asservissement MPPT opérant en fonction du rapport cyclique direct α = t_{C}/T_{P}, la figure représentant les variations de la tension de sortie Vₒᵤₜ délivrée par le PEH et de la puissance correspondante Pₒᵤₜ extraite, en fonction des variations du rapport cyclique direct α.

Il s'agit d'asservir le système sur une valeur de rapport cyclique optimal αₒₚₜ déterminée à l'avance, qui est par exemple comprise dans une plage de 50 à 55 % pour une interface de type FBR ou FBR-SO, ou de 50 à 60 % pour une interface de type SSHI, comme expliqué plus haut. Lorsque la valeur instantanée courante du rapport cyclique α, telle que mesurée par le signal V_{DUTY}, est supérieure à la valeur optimale αₒₚₜ, l'asservissement désactive le convertisseur 54, ce qui a pour effet de permettre à la tension de sortie V_{DC} d'accroître la charge électrique du condensateur intermédiaire 50. Cette situation correspond à un point de fonctionnement situé Figure 18 dans la zone DIS (*disabled*), à droite du point de fonctionnement optimal αₒₚₜ.

Inversement, lorsque la valeur courante instantanée du rapport cyclique est inférieure à la valeur optimale αₒₚₜ, le convertisseur 54 est activé pour permettre l'extraction de l'énergie accumulée dans le condensateur intermédiaire 50, et le transfert de cette énergie à la microbatterie 44 et aux divers circuits applicatifs du dispositif. Cette situation correspond à un point de fonctionnement situé Figure 18 dans la zone EN (*enabled*), à gauche du point de fonctionnement optimal αₒₚₜ.

L'extraction d'énergie du condensateur intermédiaire 50 par le convertisseur 54 va provoquer une augmentation progressive du rapport cyclique α et une diminution de la tension de sortie Vₒᵤₜ du PZT ; lorsque α franchit la limite αₒₚₜ, le convertisseur est alors désactivé (flèche A) avec des effets inverses et, par voie de conséquence, maintien du rapport cyclique, et donc de la puissance Pₒᵤₜ extraite de la lame, autour du point de fonctionnement optimal.

La Figure 19 est homologue de la Figure 18, pour des variations du rapport cyclique inverse β = t_{R}/T_{P}.

Cette hypothèse correspond au cas où le comparateur aux bornes de la diode 60 est configuré pour produire un signal haut lorsque la diode n'est pas conductrice. Le mode opératoire de l'asservissement MPPT est comparable à celui exposé plus haut, *mutatis mutandis*, pour un maintien du point de fonctionnement autour de la valeur optimale βₒₚₜ.

Les Figures 20, 22 et 24 illustrent trois exemples de circuiterie PEH mettant en oeuvre les enseignements de l'invention exposée ci-dessus, avec une boucle d'asservissement MPPT pilotée par les variations du rapport cyclique.

La Figure 20 illustre un premier exemple de circuiterie PEH, avec une boucle d'asservissement MPPT pilotée par les variations du rapport cyclique inverse β. La Figure 21 illustre les variations instantanées de divers signaux produits par cette circuiterie au cours de deux oscillations successives du PZT.

Le PZT 24 délivre une tension variable V_{Cp} (premier chronogramme de la Figure 21) qui est appliquée au circuit d'interface 52 qui est de l'un des types décrits plus haut (FBR, FBR-SO, P-SSHI) ou autre ; la tension alternative V_{AC} délivrée en sortie de ce circuit est redressée par la diode 60 pour délivrer une tension redressée V_{DC} appliquée au condensateur tampon intermédiaire 50.

L'étage régulateur MPPT 56 comprend le comparateur 62, dont les entrées respectives sont reliées aux bornes de la diode 60 et qui, comme décrit plus haut en référence à la Figure 7, délivre en sortie un signal V_{COMP} (deuxième chronogramme de la Figure 21) appliqué au filtre passe-bas LPF 64 pour donner le signal filtré V_{DUTY} (troisième chronogramme de la Figure 21) représentatif des variations du rapport cyclique des périodes de conduction aux bornes de la diode 60. Dans la. configuration illustrée, le comparateur 62 délivre une sortie au niveau haut lorsque la diode n'est pas passante, de sorte que le signal V_{DUTY} est un signal représentatif du rapport cyclique inverse β (si l'on souhaite un signal représentatif du rapport cyclique direct α il suffit d'inverser les entrées du comparateur 62).

Le signal V_{DUTY} est appliqué à l'une des entrées d'un second comparateur 80 dont l'autre entrée est reliée à un générateur 82 de tension de valeur de seuil V_{TH}. La sortie du comparateur 80 est appliquée à l'entrée d'activation EN (*enable*) du convertisseur 54. Ce convertisseur 54, lorsqu'il est activé, convertit la tension aux bornes du condensateur 50, appliquée sur l'entrée E, en une tension régulée, délivrée sur la sortie S, permettant d'assurer par un courant Iₒᵤₜ (quatrième chronogramme de la Figure 21) la recharge de la microbatterie 44 et l'alimentation des divers circuits applicatifs de l'implant situés en aval.

Le fonctionnement de cet asservissement est le suivant : en début de cycle, la tension aux bornes du condensateur 50 est relativement basse et le rapport cyclique inverse β est relativement faible. Après quelques cycles de vibration du PZT, cette tension augmente et les β diminuent : cf. les zones référencées ① et ③ sur les Figures 19 et 21.

Lorsque β atteint un seuil prédéterminé (β = βₒₚₜ + Δ, avec une valeur Δ de 5 à 10 %), correspondant au franchissement de la référence V_{TH} par le signal V_{DUTY} (comme illustré sur le troisième chronogramme de la Figure 21), alors le convertisseur 54 est activé (zone référencée ② sur les Figures 19 et 21).

L'activation du convertisseur 54 a pour effet d'extraire les charges accumulées par le condensateur 50, ce qui a pour conséquence de diminuer la tension aux bornes de celui-ci (flèche A sur la Figure 19 et zone référencée ④ sur la Figure 21). L'énergie extraite du condensateur, appliquée à l'entrée E du convertisseur 54, est régulée et transférée sur la sortie S à la microbatterie 44 et aux circuits applicatifs en aval (zone ⑥ sur la Figure 21). L'activation du convertisseur 54 dure jusqu'à ce que le rapport cyclique β redescende au-dessous de la valeur de seuil (V_{DUTY} < V_{TH}). Le convertisseur 54 est alors désactivé, ce qui permet de recommencer un cycle de charge du condensateur 50, avec corrélativement une augmentation progressive du rapport cyclique β, proche de sa valeur de seuil.

Contrairement aux indications habituelles de la littérature qui, afin de disposer d'une tension stable en sortie du convertisseur, prescrivent d'utiliser pour le condensateur tampon un composant de valeur très supérieure à la capacité interne du PZT (au moins 100 à 1000 fois supérieure), dans le cas de la présente invention il est préférable d'utiliser un condensateur tampon de faible valeur - typiquement 10 à 20 fois seulement la valeur de la capacité interne du PZT - pour pouvoir assurer un asservissement rapide de la boucle MPPT qui maximise la tension de sortie et, de fait, la puissance extraite du PZT.

Le circuit de la Figure 20 décrit précédemment permet de réaliser un asservissement de la puissance extraite du PEH sans pour autant estimer directement cette puissance. Il est parfois souhaitable de fournir un indicateur corrélé avec la puissance et utiliser un circuit MPPT préexistant pour réaliser l'asservissement. Les Figure 22 et 24 décrivent une modification du circuit de la Figure 20 pour fournir un tel indicateur de la puissance extraite.

La Figure 22 illustre un deuxième exemple de circuiterie de PEH, adapté à un étage d'alimentation préexistant de type connu comprenant déjà un étage de recherche du maximum de puissance, notamment mettant en oeuvre un détecteur d'enveloppe.

L'étage d'asservissement MPPT 56 comprend un multiplieur analogique 84 dont l'une des entrées reçoit le signal de sortie de l'ensemble comparateur 62/filtre passe-bas 64 précédemment décrit. Cette entrée reçoit donc un signal proportionnel au produit V_{DC}.α. L'autre entrée du multiplieur est reliée à une branche comprenant un étage de clamping 86 relié en entrée à la sortie du comparateur 62 et en sortie à un second filtre passe-bas 88. Cette autre entrée du multiplieur reçoit donc un signal proportionnel à α. La sortie du multiplieur délivre un signal proportionnel à K(V_{DC.}α)α, qui est représentatif d'une estimée du second ordre autour de α = 0 de la puissance.. Ce signal peut être utilisé avec des circuits d'asservissement préexistants opérant sur la base d'un signal d'entrée de type "puissance estimé". Il peut notamment s'agir de circuits comprenant un détecteur d'enveloppe MPPT 90, recevant le signal Vₑₛₜ en sortie du multiplieur 90, associé à un comparateur MPPT 92, dont la sortie pilote l'entrée d'activation EN du convertisseur 54 (les circuits 90, 92 pouvant être, dans certains composants, déjà intégrés aux autres circuits du convertisseur 54).

La Figure 23, qui représente la puissance extraite effectivement mesurée Pₒᵤₜ et le signal d'estimée Vₑₛₜ délivré par l'étage 56, montre une excellente corrélation, au voisinage du maximum de la courbe, entre l'estimée de la puissance et la puissance réellement extraite.

La Figure 24 illustre une variante de la circuiterie PEH de la Figure 22, dans laquelle le multiplieur analogique 84 reçoit directement sur l'une de ses entrées la tension redressée Voc du condensateur tampon 50, et sur son autre entrée le signal de sortie d'un second comparateur 94 dont une entrée est reliée à l'ensemble comparateur 62/filtre passe-bas 64 et dont l'autre entrée est reliée à un générateur de tension de référence 96. La première entrée reçoit donc un signal proportionnel à V_{DC}, tandis que la seconde entrée reçoit un signal proportionnel à K(α-B), B étant un offset égal à B = ½ - 1/π = 0,182. Le multiplieur 84 délivre donc un signal Vₑₛₜ proportionnel à K(α-B) × V_{DC}, représentatif du produit I × V (courant × tension). On démontre en effet que, dans une approximation au premier ordre autour de la valeur optimale, le courant moyen délivré varie linéairement en fonction du terme α-B.

Ici encore, comme illustré sur la Figure 25 qui est homologue de la Figure 23 pour le circuit de la Figure 24, on constate au voisinage du maximum de la courbe une excellente corrélation entre la puissance réellement extraite Pₒᵤₜ et l'estimée Vₑₛₜ de cette puissance, délivrée par l'étage MPPT 56.

### Avantages procurés par l'invention

La solution de l'invention que l'on vient d'exposer présente, par rapport aux circuits qui ont déjà pu être proposés, les avantages suivants :
- régulation MPPT "gratuite", c'est-à-dire basée sur l'observation de signaux déjà disponibles sur le circuit d'interface ; il n'est donc pas nécessaire de faire fonctionner le système hors de sa zone optimale, comme dans le cas les algorithmes MPPT de type P&O ou FOC, ni d'effectuer d'opération complexe telle qu'un calcul de produit V × I ;
- rapidité de réponse : il suffit d'un seul cycle de vibration du PEH pour déterminer s'il est ou non nécessaire de modifier la tension de sortie, soit un temps de réponse de l'ordre de 25 ms pour un PZT dont la fréquence propre est de 20 Hz ; cet avantage est particulièrement précieux dans le cas d'une application à un stimulateur cardiaque, où les cycles de vibration du PZT (25 ms) sont beaucoup plus courts que les cycles cardiaques (500 à 1000 ms) à l'origine des vibrations de l'ensemble inertiel ;
- simplicité de la circuiterie, qui peut être implémentée avec des circuits analogiques simples, incorporés à un ASIC, ou bien réalisée à partir de composants disponibles commercialement COTS (*Commercial Off-The-Shelf*) avec une très faible consommation énergétique, convenant pour des applications très exigeantes du point de vue du bilan énergétique tels que les implants cardiaques ;
- maximisation de la puissance transférée du PEH au condensateur intermédiaire de façon entièrement indépendante des performances du convertisseur DC/DC monté en aval de ce condensateur intermédiaire,
- mise en oeuvre de l'invention avec toute technologie connue de convertisseur DC/DC, sans modification de ce dernier.

## Revendications

1. Un module de récupération d'énergie, comprenant :
- un ensemble pendulaire (40) soumis à des sollicitations externes appliquées au module, l'ensemble pendulaire comprenant une lame (24) élastiquement déformable en flexion suivant au moins un degré de liberté, avec une extrémité encastrée et une extrémité opposée libre couplée à une masse inertielle (26),
dans lequel la lame (24) est une lame piézoélectrique formant un transducteur mécanoélectrique apte à convertir une énergie mécanique produite par des oscillations de l'ensemble pendulaire en un signal électrique alternatif oscillant (V(t)) recueilli par des électrodes de la lame (24) ; et
- un circuit de gestion d'alimentation (42), apte à redresser et réguler le signal recueilli par les électrodes, pour délivrer en sortie une tension ou un courant continus d'alimentation stabilisés,
dans lequel le circuit de gestion d'alimentation (42) comprend :
- un circuit (52) d'interfaçage avec la lame piézoélectrique, apte à recevoir en entrée le signal électrique alternatif oscillant (V(t)) délivré par la lame piézoélectrique, et délivrer en sortie un signal redressé (V_{AC}) comprenant une série de pulsations à une fréquence multiple de la fréquence d'oscillation de l'ensemble pendulaire ;
- en sortie du circuit d'interfaçage (52), un condensateur tampon (50) apte à être chargé par les pulsations successives délivrées par le circuit d'interfaçage ;
- un convertisseur régulateur (54) apte à convertir un courant de décharge du, condensateur tampon (50) en ladite tension ou courant continus d'alimentation stabilisés ; et
- un circuit de pilotage du convertisseur régulateur (54), comprenant un étage (56) d'asservissement de type *Maximum Power-Point Tracking*, MPPT, en fonction d'une estimée d'une puissance extraite de la lame piézoélectrique,
**caractérisé en ce que** l'étage d'asservissement MPPT (56) apte à être commandé par une valeur courante (V_{DUTY}) d'un rapport cyclique, direct (α) ou inverse (β), des pulsations délivrées à une fréquence multiple de la fréquence d'oscillation de l'ensemble pendulaire en sortie du circuit d'interfaçage (52).

2. Le module de la revendication 1, dans lequel :
- le circuit de gestion d'alimentation comprend en outre une diode anti-retour (60) interposée entre le circuit d'interfaçage (52) et le condensateur tampon (50) ; et
- l'étage d'asservissement MPPT (56) comprend :
• un circuit (62, 64) de détection des périodes de conduction de la diode anti-retour (60) ;
• un circuit extracteur, apte à produire un signal (V_{DUTY}) représentatif de la valeur courante du rapport cyclique à partir des périodes de conduction et de non-conduction détectées par le circuit de détection (62, 64) ; et
• un circuit (80, 82) de comparaison de la valeur courante (V_{DUTY}) du rapport cyclique à une valeur de rapport cyclique (αₒₚₜ ; βₒₚₜ) prédéterminée.

3. Le module de la revendication 2, dans lequel l'étage d'asservissement MPPT (56) comprend en outre un circuit de commande, apte à contrôler:
- si la valeur courante (V_{DUTY}) de rapport cyclique est supérieure à la valeur de rapport cyclique optimale (αₒₚₜ ; βₒₚₜ), coupler le condensateur tampon (50) au convertisseur régulateur (54) de manière à décharger le condensateur tampon vers une entrée (E) du convertisseur régulateur (54), ou
- si la valeur courante (V_{DUTY}) de rapport cyclique est inférieure à la valeur de rapport cyclique optimale (αₒₚₜ ; βₒₚₜ), découpler le condensateur tampon (50) du convertisseur régulateur (54) de manière à permettre la poursuite de la charge du condensateur tampon par les pulsations successives délivrées par le circuit d'interfaçage (52).

4. Le module de la revendication 3, dans lequel le convertisseur régulateur (54) est un régulateur abaisseur/élévateur à découpage de type survolteur/dévolteur *buck-boost* sélectivement activable/désactivable (EN) par le circuit de commande.

5. Le module de la revendication 2, dans lequel le circuit de détection des périodes de conduction et le circuit extracteur comprennent un comparateur (62) couplé en entrée à la diode anti-retour (60), apte à détecter la polarité de la différence de potentiel (V_{DC} - V_{AC}) aux bornes de la diode anti-retour (60), et en aval du comparateur (62) un filtre passe-bas (64) apte à délivrer un signal (V_{DUTY}) représentatif de la valeur courante du rapport cyclique.

6. Le module de la revendication 2, dans lequel le circuit d'interfaçage (52) comprend un circuit redresseur double alternance, FBR, à pont de diodes ou convertisseur de tension négative NVC (*Negative Voltage Converter*) à MOSFET, et la valeur de rapport cyclique (αₒₚₜ ; βₒₚₜ) prédéterminée est comprise entre 50% et 55% en termes de rapport cyclique direct (α), ou entre 45% et 50% en termes de rapport cyclique inverse (β).

7. Le module de la revendication 2, dans lequel le circuit d'interfaçage (52) comprend un circuit FBR à commutation synchronisée de décharge, FBR-SO, et la valeur de rapport cyclique (αₒₚₜ ; βₒₚₜ) prédéterminée est comprise entre 50% et 52% en termes de rapport cyclique direct (α), ou entre 48% et 50% en termes de rapport cyclique inverse (β).

8. Le module de la revendication 2, dans lequel le circuit d'interfaçage (52) comprend un circuit FBR à commutation synchronisée d'inductance parallèle de décharge, P-SSHI, et la valeur de rapport cyclique (αₒₚₜ ; βₒₚₜ) prédéterminée est comprise entre 52% et 60% en termes de rapport cyclique direct (oc), ou entre 40% et 48 % en termes de rapport cyclique inverse (β).

9. Le module de la revendication 2, dans lequel l'étage d'asservissement MPPT (56) comprend :
- un premier comparateur (62), apte à comparer les tensions aux bornes de la diode anti-retour (60) ;
- en aval du premier comparateur (62), un filtre passe-bas (64) apte à délivrer un signal représentatif de la valeur courante du rapport cyclique inverse (β) ; et
- un second comparateur, apte à comparer le signal délivré par le filtre passe-bas (64) à une référence de tension, et délivrer en sortie un signal de pilotage du convertisseur régulateur (54).

10. Le module de la revendication 2, dans lequel l'étage d'asservissement MPPT (56) comprend :
- un premier comparateur (62), apte à comparer les tensions aux bornes de la diode anti-retour (60) ;
- en aval du premier comparateur (62), un premier filtre passe-bas (64) apte à délivrer un premier signal représentatif de la valeur courante du rapport cyclique direct (α) et de la tension délivrée par le circuit d'interfaçage (52) ;
- un trigger de Schmitt (86) apte à recevoir le signal délivré par le premier comparateur (62) ;
- en aval du trigger de Schmitt (86), un second filtre passe-bas (88) apte à délivrer un second signal représentatif de la valeur courante du rapport cyclique direct (α) ; et
- un multiplieur (84) apte à combiner les signaux respectivement délivrés par le premier (64) et le second filtres passe-bas, et délivrer en sortie un signal (Vₑₛₜ) représentatif d'une estimée de la puissance extraite de la lame piézoélectrique.

11. Le module de la revendication 2, dans lequel l'étage d'asservissement MPPT (56) comprend :
- un premier comparateur (62), comparant les tensions aux bornes de la diode anti-retour (60) ;
- en aval du premier comparateur (62), un filtre passe-bas (64) apte à délivrer un premier signal représentatif de la valeur courante du rapport cyclique et du niveau de tension délivré par le circuit d'interfaçage (52) ;
- en aval du filtre passe-bas (64), un second comparateur (94) apte à comparer le signal délivré par le filtre passe-bas (64) à une référence de tension (96), et délivrer en sortie un signal fonction, à un décalage près, de la valeur courante du rapport cyclique direct (α) ; et
- un multiplieur (84) apte à combiner le signal délivré par le second comparateur et un signal de tension (V_{DC}) aux bornes du condensateur tampon (50), et délivrer en sortie un signal (Vₑₛₜ) représentatif d'une estimée de la puissance extraite de la lame piézoélectrique.

12. Le module de la revendication 10 ou 11, comprenant en outre un détecteur d'enveloppe (90, 92) apte à recevoir en entrée le signal (Vₑₛₜ) représentatif d'une estimée de la puissance extraite de la lame piézoélectrique, délivré par le multiplieur (84).

13. Le module de la revendication 1,
dans lequel le module est incorporé à un dispositif autonome (10) logeant, dans un corps de dispositif (12) : un ensemble électronique ; ledit module de récupération d'énergie ; et un organe de stockage d'énergie (44) pour l'alimentation de l'ensemble électronique,
et dans lequel ladite tension ou ledit courant continus stabilisés délivrés par le circuit de gestion d'alimentation servent à l'alimentation de l'ensemble électronique et/ou à la recharge de l'organe de stockage d'énergie du dispositif autonome.

14. Le module de la revendication 13, dans lequel le dispositif autonome (10) est un dispositif médical actif.

15. Le module de la revendication 14,
dans lequel le dispositif médical actif est une capsule autonome implantable (10) comprenant un corps de capsule (12) muni d'un élément d'ancrage (16) à une paroi d'un organe (22) d'un patient,
et dans lequel lesdites sollicitations externes auxquelles est soumis l'ensemble pendulaire (40) du module de récupération d'énergie sont des sollicitations appliquées au corps de capsule sous l'effet de mouvements de ladite paroi et/ou de variations de débit d'un flux sanguin dans le milieu environnant.

## Patentansprüche

1. Ein Energierückgewinnungsmodul, umfassend:
- eine pendelartige Anordnung (40), die äußere Belastungen erfährt, die auf das Modul aufgebracht werden, wobei die pendelartige Anordnung ein Längsorgan (24) umfasst, das gemäß zumindest einem Freiheitsgrad elastisch biegeverformbar ist und ein eingespanntes Ende und ein gegenüberliegendes freies Ende, das an eine träge Masse (26) gekoppelt ist, aufweist,
wobei das Längsorgan (24) ein piezoelektrisches Längsorgan ist, das einen mechanisch-elektrischen Wandler bildet, der eingerichtet ist, um eine mechanische Energie, die durch Schwingungen der pendelartigen Anordnung erzeugt werden, in ein oszillierendes elektrisches Wechselsignal (V(t)) umzuwandeln, das von Elektroden des Längsorgans (24) aufgefangen wird; und
- eine Versorgungsverwaltungsschaltung (42), die eingerichtet ist, um das von den Elektroden aufgefangene Signal gleichzurichten und zu regeln, um ausgangsseitig eine stabilisierte Vorsorgungsgleichspannung oder einen stabilisierten Versorgungsgleichstrom auszugeben,
wobei die Versorgungsverwaltungsschaltung (42) umfasst:
- eine Schnittstellenschaltung (52) zum Bilden einer Schnittstelle mit dem piezoelektrischen Längsorgan, die eingerichtet ist, um eingangsseitig das oszillierende elektrische Wechselsignal (V(t)) zu empfangen, das von dem piezoelektrischen Längsorgan ausgegeben wird, und ausgangsseitig ein gleichgerichtetes Signal (V_{AC}) auszugeben, das eine Reihe von Impulsen mit einer Frequenz umfasst, die ein Mehrfaches der Oszillationsfrequenz der pendelartigen Anordnung beträgt;
- am Ausgang der Schnittstellenschaltung (52) einen Pufferkondensator (50), der eingerichtet ist, um durch die aufeinanderfolgenden Impulse, die von der Schnittstellenschaltung ausgegeben werden, geladen zu werden;
- einen Wandler-Regler (54), der einrichtet ist, um einen Entladestrom des Pufferkondensators (50) in eine stabilisierte Vorsorgungsgleichspannung oder einen stabilisierten Versorgungsgleichstrom zu wandeln; und
- eine Ansteuerschaltung zum Ansteuern des Wandler-Reglers (54), die eine Stufe (56) zur Nachführung vom Maximum Power-Point Tracking(MPPT)-Typ in Abhängigkeit von einer Schätzung einer aus dem piezoelektrischen Längsorgan extrahierten Leistung umfasst,
**dadurch gekennzeichnet, dass** die MPPT-Nachführstufe (56) eingerichtet ist, um von einem aktuellen Wert (V_{DUTY}) eines direkten Taktverhältnisses (α) oder inversen Taktverhältnisses (β) der mit einer Frequenz ausgegebenen Impulse gesteuert zu werden, die ein Mehrfaches der Oszillationsfrequenz der pendelartigen Anordnung am Ausgang der Schnittstellenschaltung (52) beträgt.

2. Das Modul nach Anspruch 1, wobei:
- die Versorgungsverwaltungsschaltung ferner eine Entladeschutzdiode (60) umfasst, die zwischen der Schnittstellenschaltung (52) und dem Pufferkondensator (50) angeordnet ist; und
- die MPPT-Nachführstufe (56) umfasst:
• eine Detektionsschaltung (62, 64) zum Detektieren der Leitungszeiten der Entladeschutzdiode (60);
• eine Extraktionsschaltung, die eingerichtet ist, um ausgehend von den Leitungs- und Nichtleitungszeiten, die von der Detektionsschaltung (62, 64) detektiert werden, ein für den aktuellen Wert des Taktverhältnisses repräsentatives Signal (V_{DUTY}) zu erzeugen; und
• eine Vergleichsschaltung (80, 82) zum Vergleichen des aktuellen Wertes (V_{DUTY}) des Taktverhältnisses mit einem vorbestimmten Taktverhältniswert (αₒₚₜ; βₒₚₜ).

3. Das Modul nach Anspruch 2, wobei die MPPT-Nachführstufe (56) ferner eine Steuerschaltung umfasst, die eingerichtet ist zum Steuern:
- wenn der aktuelle Wert (V_{DUTY}) des Taktverhältnisses größer ist als der optimale Taktverhältniswert (αₒₚₜ; βₒₚₜ), der Kopplung des Pufferkondensators (50) mit dem Wandler-Regler (54), so dass der Pufferkondensator zu einem Eingang (E) des Wandler-Reglers (54) hin entladen wird, oder,
- wenn der aktuelle Wert (V_{DUTY}) des Taktverhältnisses kleiner ist als der optimale Taktverhältniswert (αₒₚₜ; βₒₚₜ), der Entkopplung des Pufferkondensators (50) von dem Wandler-Regler (54), so dass die Fortsetzung der Ladung des Pufferkondensators durch die aufeinander folgenden Impulse, die von der Schnittstellenschaltung (52) ausgegeben werden, gestattet wird.

4. Modul nach Anspruch 3, wobei der Wandler-Regler (54) ein Abwärts-/Aufwärts-Schaltregler vom *Buck-Boost* Abwärts-/Aufwärts-Typ ist, der durch die Steuerschaltung selektiv aktivierbar/deaktivierbar (EN) ist.

5. Das Modul nach Anspruch 2, wobei die Detektionsschaltung zum Detektieren der Leitungszeiten und die Extraktionsschaltung eine Vergleichseinrichtung (62) umfassen, die eingangsseitig mit der Entladeschutzdiode (60) gekoppelt ist und eingerichtet ist, um die Polarität der Potentialdifferenz (V_{DC}-V_{AC}) an den Klemmen der Entladeschutzdiode (60) zu detektieren, und abströmseitig zu der Vergleichseinrichtung (62) ein Tiefpassfilter (64) umfassen, das eingerichtet ist, um ein für den aktuellen Wert des Taktverhältnisses repräsentatives Signal (V_{DUTY}) auszugeben.

6. Das Modul nach Anspruch 2, wobei die Schnittstellenschaltung (52) eine Zweiweg-Gleichrichter, FBR, Schaltung mit Diodenbrücke oder NVC *(Negative Voltage Converter)* Negativ-Spannungswandler mit MOSFET umfasst und der vorbestimmte Taktverhältniswert (αₒₚₜ; βₒₚₜ) zwischen 50 % und 55 % in Bezug auf das direkte Taktverhältnis (α) oder zwischen 45 % und 50 % in Bezug auf das inverse Taktverhältnis (β) beträgt.

7. Das Modul nach Anspruch 2, wobei der Schnittstellenschaltung (52) eine FBR-SO (*Synchronized Switch Harvesting FBR*) Schaltung umfasst und der vorbestimmte Taktverhältniswert (αₒₚₜ; βₒₚₜ) zwischen 50 % und 52 % in Bezug auf das direkte Taktverhältnis (α) oder zwischen 48 % und 50 % in Bezug auf das inverse Taktverhältnis (β) beträgt.

8. Das Modul nach Anspruch 2, wobei die Schnittstellenschaltung (52) eine P-SSHI (*Parallel Synchronized Switch Harvesting on Inductor FBR)* Schaltung umfasst, und der vorbestimmte Taktverhältniswert (αₒₚₜ; βₒₚₜ) zwischen 52 % und 60 % in Bezug auf das direkte Taktverhältnis (α) oder zwischen 40 % und 48 % in Bezug auf das inverse Taktverhältnis (β) beträgt.

9. Das Modul nach Anspruch 2, wobei die MPPT-Nachführstufe (56) umfasst:
- eine erste Vergleichseinrichtung (62), die eingerichtet ist, um die Spannungen an den Klemmen der Entladeschutzdiode (60) zu vergleichen;
- abströmseitig zu der ersten Vergleichseinrichtung (62) ein Tiefbassfilter (64), das eingerichtet ist, um ein für den aktuellen Wert des inversen Taktverhältnisses (β) repräsentatives Signal auszugeben; und
- eine zweite Vergleichseinrichtung, die eingerichtet ist, um das von dem Tiefbassfilter (64) ausgegebene Signal mit einer Spannungsreferenz zu vergleichen und ausgangsseitig ein Signal zum Ansteuern des Wandler-Reglers (54) auszugeben.

10. Das Modul nach Anspruch 2, wobei die MPPT-Nachführstufe (56) umfasst:
- eine erste Vergleichseinrichtung (62), die eingerichtet ist, um die Spannungen an den Klemmen der Entladeschutzdiode (60) zu vergleichen;
- abströmseitig zu der ersten Vergleichseinrichtung (62) ein erstes Tiefbassfilter (64), das eingerichtet ist, um ein für den aktuellen Wert des direkten Taktverhältnisses (α) und für die von der Schnittstellenschaltung (52) ausgegebene Spannung repräsentatives erstes Signal auszugeben;
- einen Schmitt-Trigger (86), der eingerichtet ist, um das von der ersten Vergleichseinrichtung (62) ausgegebene Signal zu empfangen;
- abströmseitig zu dem Schmitt-Trigger (86) ein zweites Tiefbassfilter (88), das eingerichtet ist, um ein für den aktuellen Wert des direkten Taktverhältnisses (α) repräsentatives zweites Signal auszugeben; und
- einen Multiplikator (84), der eingerichtet ist, um die von dem ersten Tiefbassfilter (64) bzw. dem zweiten Tiefbassfilter ausgegebenen Signale zu kombinieren und ausgangsseitig ein für eine Schätzung der aus dem piezoelektrischen Längsorgan extrahierten Leistung repräsentatives Signal (Vₑₛₜ) auszugeben.

11. Das Modul nach Anspruch 2, wobei die MPPT-Nachführstufe (56) umfasst:
- eine erste Vergleichseinrichtung (62), welche die Spannungen an den Klemmen der Entladeschutzdiode (60) vergleicht;
- abströmseitig zu der ersten Vergleichseinrichtung (62) ein Tiefbassfilter (64), das eingerichtet ist, um ein für den aktuellen Wert des Taktverhältnisses und für den von der Schnittstellenschaltung (52) ausgegebenen Spannungspegel repräsentatives erstes Signal auszugeben;
- abströmseitig zu dem Tiefpassfilter (64) eine zweite Vergleichseinrichtung (94), die eingerichtet ist, um das von dem Tiefbassfilter (64) ausgegebene Signal mit einer Spannungsreferenz (96) zu vergleichen und ausgangsseitig ein Signal auszugeben, das, abgesehen von einem Versatz, von dem aktuellen Wert des direkten Taktverhältnisses (α) abhängig ist; und
- einen Multiplikator (84), der eingerichtet ist, um das von dem zweiter Vergleichseinrichtung ausgegebene Signal und ein Signal der Spannung (V_{DC}) an den Klemmen des Pufferkondensators (50) zu kombinieren und ausgangsseitig ein für eine Schätzung der aus dem piezoelektrischen Längsorgan extrahierten Leistung repräsentatives Signal (Vₑₛₜ) auszugeben.

12. Das Modul nach Anspruch 10 11, ferner umfassend einen Hüllkurvendetektor (90, 92), der eingerichtet ist, um eingangsseitig das für eine Schätzung der aus dem piezoelektrischen Längsorgan extrahierten Leistung repräsentatives Signal (Vₑₛₜ), zu empfangen, das von dem Multiplikator (84) ausgegeben wird..

13. Das Modul nach Anspruch 1,
wobei das Modul in eine autonome Vorrichtung (10) eingebettet ist, die, in einem Vorrichtungskörper (12), eine elektronische Anordnung; das Energierückgewinnungsmodul; und ein Energiespeicherorgan (44) für die Versorgung der elektronischen Anordnung aufnimmt,
und wobei die stabilisierte Gleichspannung oder der stabilisierte Gleichstrom, die/der von der Versorgungsverwaltungsschaltung ausgegeben wird, der Versorgung der elektronischen Anordnung und/oder der Aufladung des Energiespeicherorgans der autonomen Vorrichtung dienen.

14. Das Modul nach Anspruch 13, wobei die autonome Vorrichtung (10) eine aktive medizinische Vorrichtung ist.

15. Modul nach Anspruch 14,
wobei die aktive medizinische Vorrichtung eine implantierbare autonome Kapsel (10) ist, die einen Kapselkörper (12) umfasst, der mit einem Element (16) zur Verankerung an einer Wand eines Organs (22) eines Patienten versehen ist,
und wobei die äußeren Belastungen, welche die pendelartige Anordnung (40) des Energierückgewinnungsmoduls erfährt, Belastungen sind, die auf den Kapselkörper unter der Einwirkung von Bewegungen der Wand und/oder von Veränderungen einer Blutflussrate im umgebenden Medium aufgebracht werden.

## Claims

1. An energy harvesting module, comprising:
- a pendular unit (40) subjected to external stresses applied to the module, the pendular unit comprising a beam (24) that is elastically deformable in bending according to at least one degree of freedom, with a clamped end and an opposite free end coupled to an inertial mass (26),
wherein the beam (24) is a piezoelectric beam forming a mechanical-electrical transducer adapted to convert a mechanical energy produced by oscillations of the pendular unit into an oscillating alternating electrical signal (V(t)) collected by electrodes of the beam (24); and
- a power management circuit (42), adapted to rectify and regulate the signal collected by the electrodes, for outputting a stabilized power supply direct voltage or current,
wherein the power management circuit (42) comprises:
- an interface circuit (52) coupled to the piezoelectric beam, adapted to receive as an input the oscillating alternating electrical signal (V(t)) provided by the piezoelectric beam and to output a rectified signal (V_{AC}) comprising a sequence of pulses at a frequency equal to a multiple of the oscillation frequency of the pendular unit;
- at the output of the interface circuit (52), a buffer capacitor (50) adapted to be charged by the successive pulses provided by the interface circuit;
- a converter regulator (54) adapted to convert a discharge current of the buffer capacitor (50) into said stabilized power supply direct voltage or current; and
- a circuit for controlling the converter regulator (54), comprising a feedback control stage (56) of the Maximum Power-Point Tracking, MPPT, type, based on an estimate of a power extracted from the piezoelectric beam,
**characterized in that** the MPPT feedback control stage (56) is adapted to be controlled by a current value (V_{DUTY}) of a direct (α) or reverse (β) duty cycle of the pulses outputted at a frequency equal to a multiple of the oscillation frequency of the pendular unit at the output of the interface circuit (52).

2. The module of claim 1, wherein:
- the power management circuit further comprises a blocking diode (60) interposed between the interface circuit (52) and the buffer capacitor (50), and
- the MPPT feedback control stage (56) comprises:
• a circuit (62, 64) for detecting conduction periods of the blocking diode (60);
• an extraction circuit adapted to produce a signal (V_{DUTY}) representative of a current value of the duty cycle based on conduction and non-conduction periods detected by the detection circuit (62, 64); and
• a circuit (80, 82) for comparing the current value (V_{DUTY}) of the duty cycle with a predetermined duty cycle value (αₒₚₜ ; βₒₚₜ).

3. The module of claim 2, wherein the MPPT feedback control stage (56) further comprises a control circuit adapted to control:
- if the current duty cycle value (V_{DUTY}) is higher than the optimum duty cycle value (αₒₚₜ ; βₒₚₜ), coupling the buffer capacitor (50) to the converter regulator (54) so as to discharge the buffer capacitor towards an input (E) of the converter regulator (54), or
- if the current duty cycle value (V_{DUTY}) is lower than the optimum duty cycle value (αₒₚₜ ; βₒₚₜ), uncoupling the buffer capacitor (50) from the converter regulator (54) so as to allow the charging of the buffer capacitor by the successive pulses outputted by the interface circuit (52) to continue.

4. The module of claim 3, wherein the converter regulator (54) is a step-up/step-down switching regulator of the buck-boost type, which can be selectively enabled/disabled (EN) by the control circuit.

5. The module of claim 2, wherein the conduction period detection circuit and the extraction circuit comprise a comparator (62) coupled at an input to the blocking diode (60), adapted to detect the polarity of the potential difference (V_{DC} - V_{AC}) across the blocking diode (60) and, downstream from the comparator (62), a low-pass filter adapted to output a signal (V_{DUTY}) representative of the current duty cycle value.

6. The module of claim 2, wherein the interface circuit (52) comprises a Full-Bridge Rectifier, FBR, circuit, with a diode bridge or a MOSFET-based Negative Voltage Converter, NVC, and the predetermined duty cycle value (αₒₚₜ ; βₒₚₜ) is between 50% and 55% in terms of direct duty cycle (α), or between 45% and 50% in terms of reverse duty cycle (β).

7. The module of claim 2, wherein the interface circuit (52) comprises a synchronized discharge FBR-Switch Only, FBR-SO, circuit, and the predetermined duty cycle value (αₒₚₜ ; βₒₚₜ) is between 50% and 52% in terms of direct duty cycle (α), or between 48% and 50% in terms of reverse duty cycle (β).

8. The module of claim 2, wherein the interface circuit (52) comprises a Parallel Synchronized Switch Harvesting on Inductor, P-SSHI, FBR circuit and the predetermined duty cycle value (αₒₚₜ ; βₒₚₜ) is between 52% and 60% in terms of direct duty cycle (α), or between 40% and 48% in terms of reverse duty cycle (β).

9. The module of claim 2, wherein the MPPT feedback control stage (56) comprises:
- a first comparator (62) adapted to compare voltages across the blocking diode (60);
- downstream from the first comparator (62), a low-pass filter (64) adapted to output a signal representative of a current value of the reverse duty cycle (β); and
- a second comparator adapted to compare a signal output by the low-pass filter (64) with a voltage reference, and to output a signal for driving the converter regulator (54).

10. The module of claim 2, wherein the MPPT feedback control stage (56) comprises:
- a first comparator (62) adapted to compare voltages across the blocking diode (60);
- downstream from the first comparator (62), a first low-pass filter (64) adapted to output a first signal representative of a current value of the direct duty cycle (α) and of a voltage outputted by the interface circuit (52);
- a Schmitt trigger (86) adapted to receive the signal outputted by the first comparator (62);
- downstream from the Schmitt trigger (86), a second low-pass filter (88) adapted to output a second signal representative of a current value of the direct duty cycle (α); and
- a multiplier (84) adapted to combine the signals outputted by the first (64) and the second low-pass filters, respectively, and to output a signal (Vₑₛₜ) representative of an estimate of a power extracted from the piezoelectric beam.

11. The module of claim 2, wherein the MPPT feedback control stage (56) comprises:
- a first comparator (62) comparing voltages across the blocking diode (60);
- downstream from the first comparator (62), a low-pass filter (64) adapted to output a first signal representative of a current duty cycle value and of a level of a voltage outputted by the interface circuit (52);
- downstream from the low-pass filter (64), a second comparator (94) adapted to compare the signal outputted by the low-pass filter (64) with a voltage reference (96), and to output a signal that is function, but with an offset, of a current value of the direct duty cycle (α); and
- a multiplier (84) adapted to combine the signal outputted by the second comparator and a voltage signal (V_{DC}) across the buffer capacitor (50), and to output a signal (Vₑₛₜ) representative of an estimate of a power extracted from the piezoelectric beam.

12. The module of claim 10 or 11, further comprising an envelope detector (90, 92) adapted to receive as an input the signal (Vₑₛₜ) representative of an estimate of the power extracted from the piezoelectric beam, outputted by the multiplier (84).

13. The module of claim 1,
wherein the module is incorporated to an autonomous device (10) including within a device body (12): an electronic unit; said power harvesting module; and an energy storage component (44) for powering the electronic unit,
and wherein said stabilized direct voltage or current provided by the power management circuit is used to power the electronic unit and/or to charge the energy storage component of the autonomous device.

14. The module of claim 13, wherein the autonomous device (10) is an active medical device.

15. The module of claim 14,
wherein the active medical device is an implantable autonomous capsule (10) comprising a capsule body (12) provided with an anchoring element (16) for anchoring it to a wall of a patient's organ (22),
and wherein said external stresses to which is subjected the pendular unit (40) of the energy harvesting module are stresses applied to the capsule body under the effect of movements of said wall and/or blood flow rate variations in the surrounding environment.
